# EUROPÄISCHE PATENTSCHRIFT

(11) **EP 2 491 120 B1**
(45) Veröffentlichungstag und Bekanntmachung des Hinweises auf die Patenterteilung: **23.12.2015**
(21) Anmeldenummer: 10773880.9
(22) Anmeldetag: 18.10.2010
(51) Int. Cl.: C12N 9/16

(54) **SYNTHETISCHE PHYTASEVARIANTEN**
SYNTHETIC PHYTASE VARIANTS
VARIANTES DE PHYTASE SYNTHETIQUES

(30) Priorität: 22.10.2009 EP 09173782
(43) Veröffentlichungstag der Anmeldung: 29.08.2012
(73) Patentinhaber: BASF SE, 67056 Ludwigshafen (DE)
(72) Erfinder: HAEFNER, Stefan, 67346 Speyer (DE); SERWE, Annegret, 67067 Ludwigshafen (DE); STOSIK, Beata, 68219 Mannheim (DE)
(86) Internationale Anmeldenummer: PCT/EP2010/065624
(87) Internationale Veröffentlichungsnummer: WO 2011/048046

(56) Entgegenhaltungen:
- WO-A2-2010/034835
- DATABASE Geneseq [Online] 27. November 2008 (2008-11-27), "Hafnia alvei phytase protein sequence, SEQ ID 10.", XP002617492, gefunden im EBI accession no. GSP:ATR76755 Database accession no. ATR76755 -& WO 2008/116878 A1 (NOVOZYMES AS [DK]) 2. Oktober 2008 (2008-10-02) in der Anmeldung erwähnt
- DATABASE Geneseq [Online] 27. November 2008 (2008-11-27), "Hafnia alvei phytase coding sequence, SEQ ID 9.", XP002617493, gefunden im EBI accession no. GSN:ATR76754 Database accession no. ATR76754
- DATABASE Geneseq [Online] 2. Oktober 2008 (2008-10-02), "Obesumbacterium proteus phytase", XP002617494, gefunden im EBI accession no. GSP:ASS02725 Database accession no. ASS02725 -& WO 2008/092901 A2 (NOVOZYMES AS [DK]) 7. August 2008 (2008-08-07)
- LIM, B. L. ET AL.: "Distribution and diversity of phytate-mineralizing bacteria", THE ISME JOURNAL, Bd. 1, 7. Juni 2007 (2007-06-07), Seiten 321-330, XP002617495,
- ZININ N V ET AL: "Gene cloning, expression and characterization of novel phytase from Obesumbacterium proteus", FEMS MICROBIOLOGY LETTERS, BLACKWELL PUBLISHING, AMSTERDAM, NL, Bd. 236, Nr. 2, 15. Juni 2004 (2004-06-15) , Seiten 283-290, XP002391867, ISSN: 0378-1097, DOI: DOI:10.1111/J.1574-6968.2004.TB09659.X
- LEHMANN M ET AL: "Engineering proteins for thermostability: the use of sequence alignements versus rational design and directed evolution", CURRENT OPINION IN BIOTECHNOLOGY, LONDON, GB, Bd. 12, 1. Januar 2001 (2001-01-01), Seiten 371-375, XP002982993, ISSN: 0958-1669, DOI: DOI:10.1016/S0958-1669(00)00229-9
- JERMUTUS L ET AL: "Structure-based chimeric enzymes as an alternative to directed enzyme evolution: phytase as a test case", BRAUWELT, NUERNBERG, DE, Bd. 85, Nr. 1, 23. Januar 2001 (2001-01-23), Seiten 15-24, XP004315105, ISSN: 0724-696X, DOI: DOI:10.1016/S0168-1656(00)00373-4
- BEI J ET AL: "Structure-based fragment shuffling of two fungal phytases for combination of desirable properties", JOURNAL OF BIOTECHNOLOGY, ELSEVIER SCIENCE PUBLISHERS, AMSTERDAM, NL, Bd. 139, Nr. 2, 15. Januar 2009 (2009-01-15), Seiten 186-193, XP025842473, ISSN: 0168-1656, DOI: DOI:10.1016/J.JBIOTEC.2008.08.011 [gefunden am 2008-09-10]

## Beschreibung

Die vorliegende Erfindung betrifft Phytasen, Aminosäuresequenzen kodierend Phytaseenzyme sowie Nukleotidsequenzen, die Phytasen kodieren und Verfahren zur Herstellung und Verwendung von Phytasen sowie Tierfuttermitteln enthaltend diese Phytasen.

Phosphor ist ein wesentliches Element für das Wachstum von Lebewesen. Bei der Tierproduktion muss Futter mit anorganischem Phosphor in der Regel ergänzt werden, um gute Wachstumsleistungen zu erzielen. In Getreide und Hülsenfrüchten wird Phosphor hauptsächlich in Form von Phytat gespeichert. Allerdings sind monogastrische Tiere wie Schweine, Geflügel und Fische nicht in der Lage Phytat oder Phytinsäure direkt zu absorbieren, so dass dies zu einer Phytatausscheidung führt, die eine Phosphorüberanreicherungen in Regionen bedeutet, in denen eine intensive Nutztierproduktion stattfindet. Weiterhin agiert die Phytinsäure durch Binden von Metallen wie Kalzium, Kupfer oder Zink als ein den Stoffwechsel negativ beeinflussendes Mittel in monogastrischen Tieren. Um das Phosphatdefizit dieser Tiere auszugleichen und ein ausreichendes Wachstum und eine ausreichende Gesundheit sicherzustellen, wird anorganisches Phosphat dem Tierfutter zugesetzt. Dieser Zusatz von anorganischem Phosphat ist teuer und führt zu einer weiteren Belastung der Umwelt. Durch den Einsatz einer Phytase in Tierfuttermitteln wird das Phytat hydrolysiert und ergibt einen niedrigeren Gehalt an Inositolphosphat und anorganischen Phosphaten in der Gülle. Der Zusatz von Phytasen zu Tierfuttermitteln verbessert die Verfügbarkeit von organischem Phosphor und reduziert die Umweltbelastung durch ausgeschiedene, an Phytat gebundene Phosphate. In der Literatur werden eine ganze Reihe von natürlichen Phytasen, sowohl pilzlichen als auch bakteriellen Ursprungs, beschrieben.

Phytasen, auch myo-inositol hexakisphosphate phosphohydrolase genannt, sind eine Klasse von Phosphatasen, die in der Lage sind mindestens einen Phosphatrest von Phytat abzuspalten.

Die EP 420 358 beschreibt allgemein die Klonierung und Expression mikrobieller Phytasen, die WO 2006/38062 beschreibt mikrobielle Phytasen, die aus *Citrobakter freundii* stammen als Zusatz zu Tierfuttermitteln, die WO 2007/112739 beschreibt Phytasen basierend auf einer natürlichen Phytase aus *Citrobakter braakii* sowie Verfahren zu ihrer Herstellung und die Verwendung in Tierfuttermitteln.

In Haefner *et al.* (Haefner S., Knietsch A., Scholten E., Braun J., Lohscheidt M. and Zelder O. (2005) Biotechnological production and application of phytases. Appl Microbiol Biotechnol 68:588-597) werden eine Vielzahl von bekannten Anwendungen von Phytasen im Bereich der Human- oder Tierernährung beschrieben. Weitere Verwendungen von Phytasen wie beispielsweise die Verwendung zur Hydrolyse von Biomasse oder Stärke bei der Bioethanolherstellung beschreibt die WO2008/097620.
Die WO 2008/116878 beschreibt eine Phytase aus *Hafnia alvei* und deren Proteinsequenz. Zinin et al. (FEMS Microbiology Letters (2004) 236:283-290) offenbart eine Phytase aus *Obesumbacterium proteus,* deren Sequenz in der UNIPROT Datenbank mit der Eingangs-Nummer Q6U677 hinterlegt ist. In den Patentanmeldungen WO 2006/043178, WO 2008/097619 und WO 2008/092901 sind Phytasen aus verschiedenen *Buttiauxella sp.* beschrieben. Zu den natürlichen Phytasen mit den derzeit höchsten spezifischen Aktivitäten zählen die natürlichen Phytasen aus *Yersinia intermedia* (WO2007/128160) und *Yersinia pestis* (WO02/048332).

Allerdings zeigen alle diese heute verfügbaren Phytasen nicht diejenigen Eigenschaften, die für die Herstellung von Tierfutterzusatzmitteln notwendig sind. Die derzeit verfügbaren Phytasen weisen keine ausreichende thermische Stabilität auf, um ohne erhebliche Verlust ihrer Aktivität bei der Herstellung von Tierfutterpellets eingesetzt zu werden. Bei der Herstellung von Tierfutterpellets wird unter hohen Temperaturen und Feuchtigkeit die Phytase zusammen mit weiteren üblichen Tierfutterbestandteilen verpresst, um insgesamt an die Nutztiere verfüttert zu werden. Bei diesem heißen und feuchten Verpressen kommt es zu erheblichen Verlusten der Phytaseaktivität. Eine Möglichkeit, diesen Aktivitätsverlust zu verhindern, ist das aufwändige Beschichten der Phytasepartikel, so dass sie gegen die Hitzewirkung geschützt werden. Dieses Beschichten der Phytasezusätze verursacht erhebliche Zusatzkosten durch die verwendeten Fette oder Polymere, die zum Beschichten eingesetzt werden.

Eine Aufgabe der vorliegenden Erfindung war es daher, eine Phytase bereit zu stellen, die eine ausreichende thermische Stabilität aufweist, so dass sie ohne zusätzliche Schutzmaßnahmen wie Beschichtung bei der Herstellung der Futtermittelpellets eingesetzt werden kann. Weiterhin war es Aufgabe der vorliegenden Erfindung, eine Phytase bereit zu stellen mit einer ausreichend hohen spezifischen Aktivität, so dass die insgesamt zuzusetzende Menge an Phytase bei der Futterherstellung möglichst gering ist. Eine weitere Aufgabe der Erfindung bestand darin eine Phytase bereit zu stellen, welche über einen weiten pH-Bereich einsetzbar ist, um in den unterschiedlichen pH-Bereichen der Verdauungstrakte unterschiedlicher Tierarten einsetzbar zu sein und auch bei Schwankungen des pH-Bereichs durch variierende Futterbestandteile ihre Aktivität beibehält.

Diese Aufgaben werden gelöst durch eine synthetische Phytase, die mindestens 90 % Identität zur Aminosäuresequenz der Sequenz ID 18 und eine erhöhte Thermostabilität gegenüber den beiden Wildtyp Phytasen aus *Yersinia mollaretii* und *Hafnia sp.* aufweist. Diese erfindungsgemäßen Phytasen verfügen über ein Temperaturoptimum von mindestens 63 °C und eine Thermostabilität von mindestens 65 °C und sind damit geeignet, um bei der Herstellung von Futtermittelpellets eingesetzt zu werden, ohne dass sie einen erheblichen Verlust ihrer Aktivität durch die heißen und feuchten Bedingungen bei der Pelletierung erleiden. Weiterhin weisen sie einen breiten pH-Bereich von über 3 pH-Einheiten auf, in dem sie mindestens 50 % ihrer relativen Aktivität beibehalten, so dass sie in einer Vielzahl von Tieren und zusammen mit unterschiedlichen Futterbestandteilen eingesetzt werden können, ohne dass es zu Einbußen ihrer Aktivität und damit zu einem erhöhten Ausscheiden des Phosphates durch die Tiere kommt. Bevorzugt weist die erfindungsgemäße synthetische Phytase mindestens 91 %, bevorzugt 92 %, insbesondere bevorzugt 93 % und vorzugsweise 94, 95, 96, 97, 98, 99 % Identität zur Aminosäuresequenz der SEQ ID 18 auf.
Die Identität zwischen zwei Proteinsequenzen oder Nukleinsäuresequenzen ist definiert als diejenige, die das Programm *needle* in der im Oktober 2009 verfügbaren Version berechnet. *Needle* ist Teil des frei verfügbaren EMBOSS Programmpakets, das von der Webseite http://emboss.sourceforge.net/ heruntergeladen werden kann. Es werden die Standardparameter verwendet: gapopen 10.0 ("gap open penalty"), gapextend 0.5 ("gap extension penalty"), datafile EBLOSUM62 (Matrix) bei Protein und datafile EDNAFULL (Matrix) bei DNA.

Gemäß einer besonderen Ausführungsform weist die synthetische Phytase an mindestens einer der Positionen ausgewählt aus der Gruppe bestehend aus Position 1, 6, 12, 17, 84, 89, 92, 109, 137, 138, 140, 142, 143, 149, 156, 202, 205, 207, 208, 209, 228, 234, 243, 247, 248, 251, 255, 256, 261, 270, 304, 314, 320, 349, 356, 373, 382, 399, 402 und 413 , bezogen auf die Position gemäß der SEQ ID 18, eine Veränderung der Aminosäure auf. Unter Veränderung wird im Sinne der vorliegenden Erfindung ein Austausch der ursprünglichen Aminosäure wie sie in der SEQ ID 18 im Sequenzprotokoll genannt wird gegen eine andere Aminosäure verstanden. Die Aminosäuren werden hierbei nach dem üblichen Einbuchstabencode benannt. Durch die Änderung einer oder mehrerer Aminosäuren ist es möglich, die Thermostabilität der synthetischen Phytase weiter zu steigern bzw. den optimalen pH-Bereich zu verbreitern oder die spezifische Aktivität zu erhöhen.

Mit Vorteil weist die synthetische Phytase mindestens 5 Veränderungen in der Aminosäuresequenz bezogen auf die SEQ ID 18 auf, insbesondere weist sie mindestens 10, mindestens 12, mindestens 14, mindestens 16, mindestens 17, mindestens 18, mindestens 19 und ganz besonders bevorzugt mindestens 20 Veränderungen auf.

Bevorzugt wird mindestens eine der Aminosäuren an einer der Positionen ausgewählt aus den Positionen 1, 6, 12, 17, 84, 89, 92, 109, 137, 138, 140, 142, 143, 149, 156, 202, 205, 207, 208, 209, 228, 234, 243, 247, 248, 251, 255, 256, 261, 270, 304, 314, 320, 349, 356, 373, 382, 399, 402 und 413, bezogen auf die Position in der SEQ ID 18, gegen eine der folgenden Aminosäuren ausgetauscht, wobei es sich vorteilhafterweise bei Position 1 um N, D, Q, H, an Position 6 um V, I, L, T, S, an Position 12 um N, D, Q, H, S, an Position 17 um N, D, Q, H an Position 84 um V, I, L, T, S, an Position 89 um T, S, V, an Position 92 um E, Q, K, R, N, P, A, S, G, H, an Position 109 um K, R, E, D, an Position 137 um L, I, V, an Position 138 um N, Q, H, S, an Position 140 um P, A, N, an Position 142 um T, S, V, an Position 143 um Y, F, W, an Position 149 um H, N, S, an Position 156 um R, K, E, D, an Position 202 um S, T, A, V, an Position 205 um R, K, E, D, an Position 207 um E, Q, D, R, N, T, S, V, A, I, L, M, an Position 208 um M, L, I, an Position 209 um S, T, an Position 228 um Y, F, W, an Position 234 um N, D, Q, H, S, I, V, L, M, an Position 243 um K, R, D, an Position 247 um K, R, E, an Position 248 um L, I, M, V, an Position 251 um N, D, Q, H, S, I, V, L, M, an Position 255 um V, I, L, T, S, an Position 256 um Y, F, W, H, N, S, an Position 261 um E, Q, D, K, R, N, an Position 270 um K, R, E, D, an Position 304 um V, I, L, T, S, an Position 314 um G, A, an Position 320 um L, I, M, V, an Position 349 um R, K, E, D, an Position 356 um L, I, M, V, an Position 373 um I, V, L, M, an Position 382 um G, A, an Position 399 um I, V, L, M, an Position 402 um N, D, Q, H, S und an Position 413 um L, I, M, V, Q, E, N, K, R als neu eingeführte Aminosäure handelt.

In einer bevorzugten Ausführungsform weist die synthetische Phytase mindestens eine der folgenden Veränderungen gegenüber der Aminosäuresequenz der SEQ ID 18 auf: S1 N; A6V; K12N; S17N; A84V; A89T; D92E; D92P; D92A D92N; Q109K; M137L; D138N; S140P; A142T; H143Y; Q149H; T156R; N202S; N202T; G205R; K207E; K207T; K207I; V208M; A209S; H228Y; K234N; K234I; E243K; D247K; S248L; K251 N; K251I; A255V; Q256Y; Q256H; S261E; N270K; A304V; S314G; T320L; Q349R; F356L; S373I; E382G; T399I; K402N; H413L; H413Q.
Dabei wird die vor der jeweiligen Positionsnummer genannte Aminosäure aus der SEQ ID 18 gegen eine der nach der Positionsnummer genannten Aminosäuren ausgetauscht. Dabei ist jeder mögliche genannte Aminosäureaustausch mit jeder der übrigen Änderungen in Kombination möglich.

Vorteilhafterweise umfasst die synthetische Phytase aus der vorliegenden Erfindung mindesten 5 der oben genannten Veränderungen, insbesondere mindestens 10, 12, 14, 16, 17, 18, 19 und insbesondere bevorzugt 20 dieser Veränderungen.

Ganz besonders bevorzugte Ausführungsformen der synthetischen Phytase weisen eine der folgenden kumulierten Summen an Veränderungen in Bezug auf die Seq ID 18 aus:
a) A89T / D92A / H143Y / N202S / K207E / A209S / H228Y / K234I / K251N / Q256H / H413Q
b) A89T / D92N / A142T / H143Y / N202S / K207E / A209S / H228Y / K234I / D247K / K251 N / Q256H / F356L / H413Q
c) A89T / D92A / H143Y / T156R / N202S / K207E / A209S / H228Y / K234I / K251 N / Q256H / S314G / H413Q oder
d) A89T / D92A / A142T / H143Y / N202S / K207E / A209S / H228Y / K234I / D247K / K251 N / Q256H / H413Q

Die jeweiligen einzelnen oder kumulativen Mutationen können je nach Position und ausgetauschter Aminosäure eine Erhöhung der Thermostabilität der synthetischen Phytase um 1 bis 11 °C bewirken, so dass eine entsprechend der jeweiligen Anwendung gewünschte Thermostabilität der Phytase durch die Auswahl der entsprechenden Anzahl und Art der Mutationen ausgewählt werden kann.

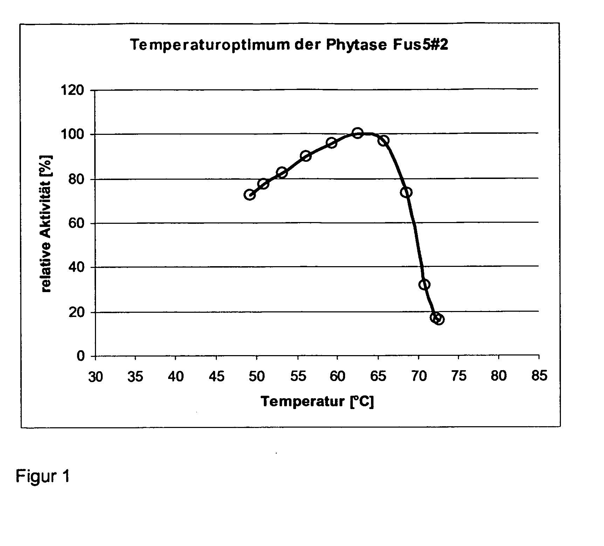

Diese besonders bevorzugten kumulierten Mutationen der synthetischen Phytase mit Nr. A-518, A-521, A-534 und A-519 (Definitionen siehe Tabelle 1) ergeben jeweils eine Erhöhung der Thermostabilität um mindestens 10 °C gegenüber der synthetischen Phytase der SEQ ID 18 (Fus5#2). Damit ergeben sich für diese besonders bevorzugten Ausführungsformen Thermostabilitäten von mindestens 10 °C über den 65 °C, die bereits für die Phytase Fus5#2 (SEQ ID 18) erzielt wurde. Das Temperaturprofil, das pH-Profil und die Thermostabilität der Phytase der SEQ ID 18 ist jeweils in den Figuren 1, 2 und 3 dargestellt.

In einer Ausführungsform weist die synthetische Phytase mindestens einen konservativen Aminosäureaustausch an den genannten Positionen gegenüber einer der folgenden oben beschriebenen Phytasen auf:
S1N; A6V; K12N; S17N; A84V; A89T; D92E; D92P; D92A D92N; Q109K; M137L; D138N; S140P; A142T; H143Y; Q149H; T156R; N202S; N202T; G205R; K207E; K207T; K207I; V208M; A209S; H228Y; K234N; K234I; E243K; D247K; S248L; K251 N; K251I; A255V; Q256Y; Q256H; S261E; N270K; A304V; S314G; T320L; Q349R; F356L; S373I; E382G; T399I; K402N; H413L; H413Q;
A89T / D92A / H143Y / N202S / K207E / A209S / H228Y / K234I / K251N / Q256H / H413Q;
A89T / D92N / A142T / H143Y / N202S / K207E / A209S / H228Y / K234I / D247K / K251 N / Q256H / F356L / H413Q;
A89T / D92A / H143Y / T156R / N202S / K207E / A209S / H228Y / K234I / K251N / Q256H / S314G / H413Q und
A89T / D92A / A142T / H143Y / N202S / K207E / A209S / H228Y / K234I / D247K / K251 N / Q256H / H413Q bezogen auf die Seq ID 18,
wobei die synthetische Phytase mindestens eine der einzelnen genannten Veränderungen oder eine der genannten Gruppen an Veränderungen aufweisen kann. Konservativ im Sinne der vorliegenden Erfindung bedeutet ein Austausch der Aminosäure G zu A; A zu G, S; V zu I,L,A,T,S; I zu V,L,M; L zu I,M,V; M zu L,I,V; P zu A,S,N; F zu Y,W,H; Y zu F,W,H; W zu Y,F.H; R zu K,E,D; K zu R,E,D; H zu Q,N,S; D zu N,E,K,R,Q; E zu Q,D,K,R,N; S zu T,A; T zu S,V,A; C zu S,T,A; N zu D,Q,H,S; Q zu E,N,H,K,R. Dabei ist es möglich, jeden konservativen Austausch einer Aminosäure mit jedem konservativen Austausch einer anderen Aminosäure zu kombinieren.

Vorteilhafterweise handelt es sich bei der synthetischen Phytase um eine isolierte Phytase. Es ist auch denkbar, dass die synthetische Phytase nicht als aufgereinigte isolierte Phytase vorliegt, sondern als Fermentationsbrühe, wobei die Biomasse ganz, teilweise oder gar nicht abgetrennt ist. Dabei kann die Brühe durch Flüssigkeitsentzug eingeengt oder vollständig getrocknet werden. Es ist möglich, diese ungereinigten oder teilgereinigten Phytase Lösungen oder Feststoffe als Zusatz in unterschiedlichen Produkten einzusetzen.

Die erfindungsgemäße synthetische Phytase weist mit Vorteil eine erhöhte spezifische Aktivität gegenüber den beiden Wildtypphytasen aus den Organismen *Yersinia mollaretii* und *Hafnia sp.,* die Grundlage der Konstruktion des synthetischen Phytasekonstruktes gemäß der SEQ ID 18 waren, auf.

Die Erfindung umfasst weiterhin eine isolierte Nukleinsäure, die eine der erfindungsgemässen Phytasen gemäß der vorgehenden Beschreibung mit den genannten möglichen Veränderungen an einzelnen oder mehreren Positionen, insbesondere eine Phytase codiert mit Veränderungen an folgenden Aminosäure Positionen bezogen auf die Seq ID 18:
a) A89T / D92A / H143Y / N202S / K207E / A209S / H228Y / K234I / K251N / Q256H / H413Q oder
b) A89T / D92N / A142T / H143Y / N202S / K207E / A209S / H228Y / K234I / D247K / K251 N / Q256H / F356L / H413Q oder
c) A89T / D92A / H143Y / T156R / N202S / K207E / A209S / H228Y / K234I / K251N / Q256H / S314G / H413Q oder
d) A89T / D92A / A142T / H143Y / N202S / K207E / A209S / H228Y / K234I / D247K / K251 N / Q256H / H413Q.

Die Erfindung umfasst ebenfalls eine isolierte Nukleinsäuresequenz, die ein Enzym mit Phytaseaktivität und einer erhöhten Thermostabilität gegenüber den beiden Wildtyp Phytasen aus *Yersinia mollaretii* und *Hafnia sp.* kodiert, wobei die Nukleinsäuresequenz mindestens 90 % Identität zur Nukleinsäuresequenz der SEQ ID 19 aufweist oder eine Nukleinsäuresequenz, die mit dem komplementären Strang einer der oben genannten Sequenzen mit mindestens 90 % Identität zur Nukleinsäuresequenz der SEQ ID 19 unter hochstringenten Bedingungen hybridisiert. In einer besonderen Ausführungsform weist die isolierte Nukleinsäuresequenz mehr als 90 % Identität, insbesondere 91, 92, 93, 94, 95, 96, 97, 98, 99 % Identität zur Seq ID 19 auf.

Die Erfindung umfasst weiterhin einen rekombinanten Expressionsvektor enthaltend eine der erfindungsgemäßen Nukleinsäuresequenzen.

Die Erfindung umfasst ebenfalls eine rekombinante Wirtszelle enthaltend eine der erfindungsgemäßen Nukleinsäuren oder enthaltend den erfindungsgemäßen rekombinanten Expressionsvektor.

Die Aufgabe wird weiterhin gelöst durch einen rekombinanten Produktionsorganismus, wobei es sich um einen nicht-menschlichen Produktionsorganismus handelt, der eine der erfindungsgemäßen Nukleinsäuresequenzen enthält oder der den erfindungsgemäßen rekombinanten Expressionsvektor enthält. Besonders bevorzugt handelt es sich bei dem rekombinanten Produktionsorganismus um einen aus der Gattung *Aspergillus, Pichia, Trichoderma, Hansenula, Saccharomyces, Bacillus, Escherischia, Kluyveromyces, Schizosaccharomyces.*

Die Aufgabe wird weiterhin gelöst durch einen Tierfuttermittelzusatz, der mindestens eine der erfindungsgemäßen Phytasen sowie weitere übliche Futtermittelzusatzstoffe, beispielsweise für Rinder, Geflügel oder Schweine, wie beispielsweise Vitamine, Mineralstoffe oder andere Zusatzstoffe enthält.

Die Aufgabe wird weiterhin gelöst durch ein Tierfuttermittel, das mindestens eine der beschriebenen erfindungsgemäßen synthetischen Phytasen enthält zusammen mit üblichen Tierfutterbestandteilen. Hierbei sind alle Futterbestandteile, wie sie üblicherweise bei Futterpellets für die Rinder-, Milchkuh-, Geflügel- oder Schweinemast eingesetzt werden, denkbar.

Die Erfindung wird weiterhin gelöst durch die Verwendung einer der beschriebenen erfindungsgemäßen synthetischen Phytasen oder des erfindungsgemäßen Tierfuttermittelzusatzes enthaltend mindestens eine der erfindungsgemäßen synthetischen Phytasen in einem Tierfuttermittel. Dabei kann die Verwendung in Form des Zusatzes der erfindungsgemäßen Phytase oder des erfindungsgemäßen Tierfuttermittelzusatzes vor der Pelletierung der übrigen Futtermittelbestandteile erfolgen. Es ist auch denkbar, dass die Phytase nach der Herstellung von Futtermittelpellets auf diese Pellets aufgebracht wird, insbesondere in flüssiger Form.

Die Erfindung wird weiterhin gelöst durch Verwendung einer der oben beschriebenen erfindungsgemäßen synthetischen Phytasen, des erfindungsgemäßen Tierfuttermittelzusatzes, welcher mindestens eine der erfindungsgemäßen synthetischen Phytasen enthält oder des Tierfuttermittels, welches mindestens eine der beschriebenen synthetischen Phytasen enthält, zur Reduktion des Phosphatgehalts in der Gülle von Nutztieren.

Die beschriebenen Ausführungsformen dienen zur Erläuterung und zum besseren Verständnis der Erfindung und sind in keiner Weise einschränkend zu verstehen. Weitere Merkmale der Erfindung ergeben sich aus der nachfolgenden Beschreibung von bevorzugten Ausführungsformen in Verbindung mit den Unteransprüchen. Hierbei können die einzelnen Merkmale der Erfindung bei einer Ausführungsform jeweils einzeln oder zu mehreren verwirklicht sein und stellen in keiner Weise eine Beschränkung der Erfindung auf die beschriebene Ausführungsform dar. Der Wortlaut der Patentansprüche wird hiermit ausdrücklich zum Gegenstand der Beschreibung gemacht.

### Figurenbeschreibung

Figur 1 zeigt das Temperaturprofil der Phytase Fus5#2. Die Phytaseaktivität wird bei der jeweils angegebenen Temperatur bestimmt. Für die Ermittlung der relativen Aktivitätswerte wird die höchste gemessene Aktivität auf 100 % gesetzt.
Figur 2 zeigt das pH Profil der Phytase Fus5#2. Die Phytaseaktivität wird bei dem jeweils angegebenen pH bestimmt. Für die Ermittlung der relativen Aktivitätswerte wird die höchste gemessene Aktivität auf 100 % gesetzt.
Figur 3 zeigt die Temperaturstabilität der Phytase Fus5#2. Die Phytase wird bei pH 5,5 für 20 min auf die angegebene Temperatur erhitzt. Nach dem Abkühlen wird die Restaktivität bei pH 5,5 und 37 °C ermittelt. Für die Ermittlung der relativen Restaktivität wird die Aktivität einer Referenzprobe, die 20 min bei Raumtemperatur inkubiert, auf 100 % gesetzt.

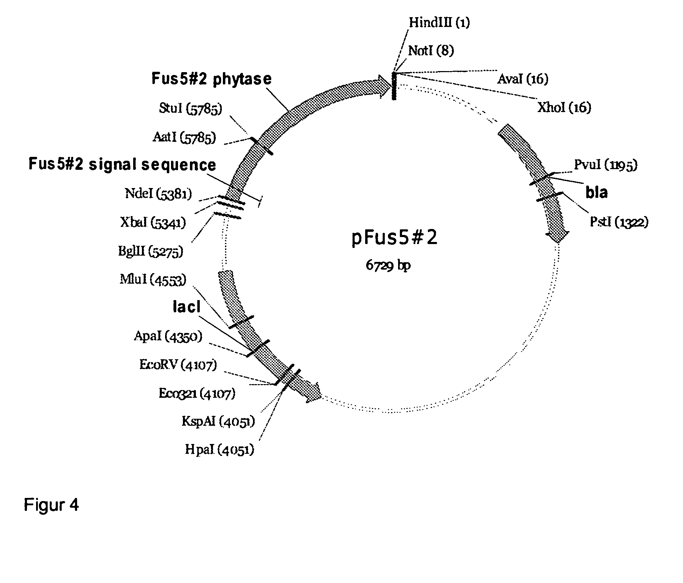

Figur 4 zeigt die Plasmidkarte des Expressionsplasmids pFus5#2.

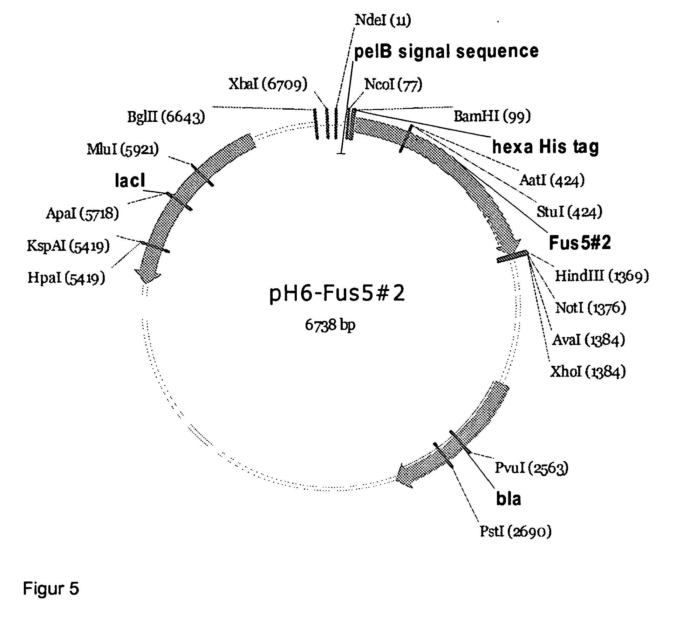

Figur 5 zeigt die Plasmidkarte des Expressionsplasmids pH6-Fus5#2.

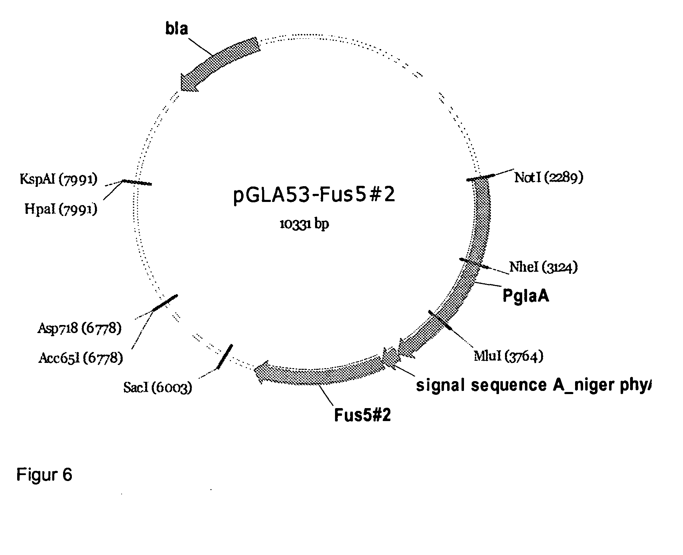

Figur 6 zeigt die Plasmidkarte des Expressionsplasmids pGLA53-Fus5#2.

### Beispiele

### Klonierung der Phytase aus Hafna sp. LU11047

In einer Reihe von Enterobakterien wird analog zu den Publikationen Huang et al. (2006) A novel phytase with preferable characteristics from Yersinia intermedia. Biochem Biophys Res Commun 350: 884-889, Shi et al. (2008) A novel phytase gene appA from Buttiauxella sp. GC21 isolated from grass carp intestine. Aquaculture 275:70-75 und WO2008116878 (Beispiel 1) mit Hilfe der degenerierten Oligos Haf1090 5'-GAYCCNYTNTTYCAYCC-3' (SEQ ID NO: 1) und Haf1092 5'-GGNGTRTTRTCNGGYTG-3' (SEQ ID NO: 2) bei Annealingtemperaturen zwischen 40 °C und 50 °C mittels PCR nach Phytasen gesucht. Die gebildeten PCR Produkte werden unter gleichen Annealingbedingungen als Template für eine Semi-Nested PCR mit den Oligos Haf1090 5'-GAYCCNYTNTTYCAYCC-3' (SEQ ID NO: 1) und Haf1091 5'-GCDATRTTNGTRTCRTG-3' (SEQ ID NO: 3) eingesetzt. Aus einem Bakterienstamm der Gattung *Hafnia* (*Hafnia sp.* LU11047) kann ein Fragment isoliert werden. Das isolierte Fragment wird mit Hilfe des "TOPO TA Cloning ® Kit" (Invitrogen) nach Angaben des Herstellers subkloniert und anschließend sequenziert. Ausgehend von dieser Teil-Sequenz wird die Volllängen Sequenz der Phytase über die sogenannte TAIL-PCR Methode (Yao-Guang Liu und Robert F. Whittier (1995) Thermal asymmetric interlaced PCR: automatable amplification and sequencing of insert end fragments from P1 and YAC clones for chromosome walking. Genomics 25, 674-681) amplifiziert. Hierzu werden folgende Oligonukleotide verwendet:
Amplifikation des 3' Endes:
   1. Haf1165 (5-WCAGNTGWTNGTNCTG-3', SEQ ID NO: 4) und
      Haf1167 (5'-CTTCGAGAGCCACTTTATTACCGTCG-3', SEQ ID NO: 5)
   2. Haf1165 (5'-WCAGNTGWTNGTNCTG-3', SEQ ID NO: 4) und
      Haf1168 (5'-CCAATGTTGTGCTGCTGACAATAGG-3', SEQ ID NO: 6)
   3. Haf1165 (5'-WCAGNTGWTNGTNCTG-3', SEQ ID NO: 4) und
      Haf1169 (5'-CCGAACTCATCAGCGCTAAAGATGC-3', SEQ ID NO: 7)

Amplifikation des 5' Endes:
1. Haf1077 (5'- CAWCGWCNGASASGAA-3', SEQ ID NO: 8) und Haf1170 (5'- CGCAGTTTGACTTGATGTCGCGCACG-3', SEQ ID NO: 9)
2. Haf1077 (5'- CAWCGWCNGASASGAA-3', SEQ ID NO: 8) und Haf1171 (5'- GTCGCGCACGCCCTATATCGCCAAGC-3', SEQ ID NO: 10)
3. Haf1077 (5'- CAWCGWCNGASASGAA-3', SEQ ID NO: 8) und Haf1172 (5'- CTGCAAACCATCGCACACGCACTGG-3', SEQ ID NO: 11)

Die erhaltenen DNA Fragmente werden mit Hilfe des "TOPO TA Cloning ® Kit" (Invitrogen) kloniert und sequenziert. Aus den Nukleotidsequenzen ergibt sich das für die Phytase aus *Hafnia sp.* LU11047 kodierende Gen SEQ ID NO: 12. Die daraus abgeleitete Aminosäuresequenz SEQ ID NO: 13 ist zu 98 % mit der Phytasesequenz aus WO2008116878 einer *Hafnia alvei* Phytase identisch.

Mit Hilfe der Software SignaIP 2.0 werden die Aminosäuren 1-33 als Signalpeptid vorhergesagt. Das gereifte Enzym beginnt demnach mit dem Serin in Position 34.

### 1. Synthetische Phytase Fus5#2

### Klonierung der Phytase Fus5#2

Ausgehend von der chromosomalen DNA aus *Hafnia sp.* LU11047 wird mittels PCR ein Fragment von Base 1-1074 der Phytase amplifiziert (SEQ ID NO: 14). Aus der DNA Sequenz einer potentiellen Phytase (bzw. sauren Phosphatase) aus *Yersinia mollaretii* ATCC43969, NCBI Sequenz ID ZP_00824387 werden Oligonukleotide für die Amplifikation der Nukleotide 1057-1323 abgeleitet. Damit wird ein zweites Phytasefragment aus der chromosomalen DNA aus *Yersinia mollaretii* ATCC 43969 amplifiziert (SEQ ID NO: 15). Bei der Amplifikation der beiden Phytasefragmente wird sowohl am 3'-Ende des *Hafnia* Fragmentes als auch am 5'-Ende des *Yersinia* Fragmentes mit Hilfe der verwendeten Oligos eine Überlappung von 20 bp zum jeweils anderen Phytasefragment erzeugt. Auf diese Weise können beide Fragmente über eine PCR Fusion zur Phytasesequenz SEQ ID NO: 16 vereinigt werden, welche für die synthetische Phytase Fus5#2 kodiert. Für die daraus abgeleitete Aminosäuresequenz SEQ ID NO: 17 werden durch die Software SignaIP 2.0 die Aminosäuren 1-33 als Signalpeptid vorhergesagt. Die reife Phytase Fus5#2 (SEQ ID NO: 18) wird durch die Nukleotidsequenz SEQ ID NO: 19 kodiert.
Für die Klonierung eines Expressionsplasmides für *E. coli* wird am 5'-Ende des Phytase DNA Fragmentes SEQ ID NO: 16 eine *Nde*I Restriktionsschnittstelle und am 3'-Ende ein Stop-Codon und eine HindIII Restriktionsschnittstelle erstellt. Die hierfür zusätzlich benötigten Sequenzen werden mittels einer PCR Reaktion über die verwendeten Primer unter Benutzung der Phytase SEQ ID NO: 16 als Template eingeführt. Unter Verwendung dieser Schnittstellen wird das für die Phytase kodierende Gen in den *E. coli* Expressionsvektor pET22b (Novagen) kloniert. Durch die Verwendung der *Nde*I Restriktionsschnittstelle und durch die Einführung des Stop Codons, wird die peIB Signalsequenz aus dem Vektor entfernt und ein Durchlesen in den auf dem Plasmid vorhandenen 6xHis Tag verhindert. Das so hergestellte Plasmid pFus5#2 (SEQ ID NO: 20) wird in den *E. coli* Stamm BL21(DE3) (Invitrogen) transformiert.
Für die verbesserte Aufreinigung des Phytaseproteins wird eine Phytase-Variante mit einem N-terminalen 6xHis Tag kloniert. Unter Verwendung des sense-Oligos PrimerH6: 5'-ctatggatccgcatcatcatcatcatcacagtgataccgcccctgc-3' (SEQ ID NO: 21), das sowohl den 6xHis Tag als auch eine *Bam*HI Schnittstelle einführt, und der für das gereifte Phytaseprotein kodierenden Sequenz SEQ ID NO: 19 als Template, wird ein PCR Produkt amplifiziert. Am 3'-Ende des PCR Produktes wird unter Verwendung des gleichen antisense Oligos wie zuvor wieder ein Stop Codon und eine *Nde*I Restriktionsschnittstelle eingeführt. Das so hergestellte Fragment wird über *Bam*HI/*Nde*I in den Vektor pET22b kloniert und das Plasmid pH6-Fus5#2 (SEQ ID NO: 22) erhalten, welches ebenfalls in *E. coli* BL21(DE3) transformiert wird. Im Falle dieses Konstruktes wird die im pET22b enthaltene pelB Signalsequenz für den Transport in das Periplasma verwendet.

### Expression der Phytase Fus5#2 in Escherichia coli

Die *E. coli* BL21(DE3) Stämme, welche ein Plasmid mit einer Phytaseexpressionkassette tragen, werden in LB Medium mit Ampicillin (100 mg/L) bei 37 °C angezogen. Die Phytaseexpression wird bei einer OD (600 nm) von 0,6 durch die Zugabe von 1 mM IPTG induziert. Nach 4 h Induktion wird 10 % (v/v) einer 10x BugBuster Lösung (Novogen) zugegeben und für 15 min bei Raumtemperatur inkubiert. Nach Zentrifugation wird der Überstand für die Bestimmung der Phytaseaktivität verwendet.

### Reinigung über Ni-Affinitätschromatographie

Für die Aufreinigung der 6xHis markierten Phytasevarianten wird eine induzierte, Phytase exprimierende *E. coli* Kulturbrühe mit 300 mM NaCl, Complete™ Protease Inhibitor ohne EDTA (nach Angaben des Herstellers Roche Applied Science) und mit 10 % (v/v) einer 10x BugBuster Lösung (Novogen) versetzt und für 15 min bei Raumtemperatur inkubiert. Nach Zentrifugation wird der Überstand an Ni-NTA Säulen/KIT (Qiagen) nach Angaben des Herstellers gebunden. Die Elution im Anschluss an die Waschschritte erfolgt mit kaltem Elutionspuffer (50 mM Na Acetatpuffer, 300 mM NaCl, 500 mM Imidazol, 1 mM CaCl₂).Vor Bestimmung des Proteingehaltes wird die Probe durch Dialyse gegen 2 mM Natriumcitrat pH 5,5 umgepuffert.

### Expression der Phytase Fus5#2 in Aspergillus niger

Für die Expression der Phytase Fus5#2 in *Aspergillus niger* wird zunächst ein Expressionskonstrukt erstellt, das das Phytase Gen unter der Kontrolle des *A. niger* Glucoamylase (glaA) Promotors, flankiert vom nicht kodierenden 3'-glaA Bereich enthält. Auf diese Weise ist das Konstrukt für eine Integration in den 3'-glaA Bereich in *A. niger* bestimmt. Als Signalsequenz für die extrazelluläre Proteinsekretion wird die Signalsequenz der *A. ficuum* Phytase verwendet. Als Basis für das Expressionskonstrukt wird das detailliert in EP0635574B1 beschriebene Plasmid pGBGLA-53 (das in WO9846772 auch als pGBTOPFYT-1 bezeichnet wird) verwendet. Mit Hilfe von dem Fachmann bekannten PCR basierten Klonierungsmethoden wird in pGBGLA-53 der Genabschnitt der *A. ficuum* Phytase, der für das gereifte Phytaseprotein beginnend mit der Aminosäuresequenz ASRNQSS kodiert, durch den für die gereifte Fus5#2 Phytase kodierenden Genabschnitt SEQ ID NO: 19 ersetzt. Es entsteht das resultierende Plasmid pGLA53-Fus5#2 (SEQ ID NO: 23). Die Co-Transformation der mit *Hind*III aus dem erhaltenen Plasmid isolierten linearen Expressionskassette gemeinsam mit einer aus dem Plasmid pGBLA50 (EP0635574B1) / pGBAAS-1 (Bezeichung des gleichen Plasmides in WO9846772) isolierten amdS Markerkassette in einen glaA deletierten *A*. *niger* Expressionsstamm und die anschließende Expression der Phytase in Schüttelkolben erfolgt wie in den beiden zitierten Patentschriften beschrieben. Die Phytaseaktivität im Kulturüberstand wird nach Abzentrifugation der Zellen täglich bestimmt. Die maximale Aktivität wird zwischen Tag 3 und Tag 6 erreicht.

### Bestimmung der spezifischen Aktivität

Die Bestimmung der Phytaseaktivität erfolgt in Mikrotiterplatten. Die gereinigte Enzymprobe wird in Reaktionspuffer (250 mM Na-Acetat, 1 mM CaCl₂, 0,01 % Tween 20, pH 5,5) verdünnt. 10 µl der Enzymlösung werden mit 110 µl Substratlösung (6 mM Na-Phytat (Sigma P3168) in Reaktionspuffer) für 20 min bei 60 °C inkubiert. Die Reaktion wird durch Zugabe von 80 µl Trichloressigsäurelösung (15 % w/w) abgestoppt. 20 µl der abgestoppten Reaktionslösung werden zur Detektion des freigesetzten Phosphates mit 280 µl frisch angesetztem Färbereagenz (60 mM L-Ascorbinsäure (Sigma A7506), 2,2 mM Ammoniummolybdat-tetrahydrat, 325 mM H₂SO₄) versetzt und für 25 min bei 50 °C inkubiert und anschließend die Absorption bei 820 nm ermittelt. Als Leerwert wird der Substratpuffer alleine bei 37 °C inkubiert und 10 µl Enzymprobe erst nach dem Abstoppen mit Trichloressigsäure hinzugegeben. Die Färbereaktion erfolgt analog. Die Menge an freigesetztem Phosphat wird über eine Kalibrierungskurve der Färbereaktion mit einer Phosphatlösung bekannter Konzentration bestimmt. Die Enzymaktivität, die unter diesen Bedingungen 1 µmol Phosphat pro min freisetzt wird als 1 U bezeichnet. Die Proteinkonzentration der eingesetzten Phytaselösung wird anhand der Absorption bei 280 nm ermittelt. Hierzu wird mit Hilfe der "Vector NTI" Software (Invitrogen, Version 10.3.0) der molekulare Extinktionskoeffizient der Phytase bestimmt.
Die spezifische Aktivität der Fus5#2 Phytase beträgt 2300 +/-200 U/mg.

### Phytaseassay

Die Bestimmung der Phytaseaktivität erfolgt in Mikrotiterplatten. Die Enzymprobe wird in Reaktionspuffer (250 mM Na-Acetat, 1 mM CaCl₂, 0,01 % Tween 20, pH 5,5) verdünnt. 10 µl der Enzymlösung werden mit 140 µl Substratlösung (6 mM Na-Phytat (Sigma P3168) in Reaktionspuffer) für 1 h bei 37 °C inkubiert. Die Reaktion wird durch Zugabe von 150 µl Trichloressigsäurelösung (15 % w/w) abgestoppt. 20 µl der abgestoppten Reaktionslösung werden zur Detektion des freigesetzten Phosphates mit 280 µl frisch angesetztem Färbereagenz (60 mM L-Ascorbinsäure (Sigma A7506), 2,2 mM Ammoniummolybdat-tetrahydrat, 325 mM H₂SO₄) versetzt und für 25 min bei 50 °C inkubiert und anschließend die Absorption bei 820 nm ermittelt. Als Leerwert wird der Substratpuffer alleine bei 37 °C inkubiert und die 10 µl Enzymprobe erst nach dem Abstoppen mit der Trichloressigsäure hinzugegeben. Die Färbereaktion erfolgt analog den restlichen Messwerten. Die Menge an freigesetztem Phosphat wird über eine Kalibrierungskurve der Färbereaktion mit einer Phosphatlösung bekannter Konzentration bestimmt.

### Bestimmung des Temperaturoptimums

Zur Bestimmung des Temperaturoptimums wird die Enzymprobe in Reaktionspuffer (250 mM Na-Acetat, 1 mM CaCl₂, 0,01 % Tween 20, pH 5,5) verdünnt. 10 µl der vortemperierten (5 min, jeweilige Reaktionstemperatur) Enzymlösung werden mit 110 µl vortemperierter Substratlösung (6 mM Na-Phytat (Sigma P3168) in Reaktionspuffer) für 30 min inkubiert. Die Inkubation erfolgt bei verschiedenen Temperaturen in einem Gradienten-Heizblock. Die Reaktion wird durch Zugabe von 80 µl Trichloressigsäurelösung (15 % w/w) abgestoppt. 20 µl der abgestoppten Reaktionslösung werden zur Detektion des freigesetzten Phosphates mit 280 µl frisch angesetztem Färbereagenz (60 mM L-Ascorbinsäure (Sigma A7506), 2,2 mM Ammoniummolybdat-tetrahydrat, 325 mM H₂SO₄) versetzt und für 25 min bei 50 °C inkubiert und anschließend die Absorption bei 820 nm ermittelt. Als Leerwert wird der Substratpuffer alleine bei der angegebenen Temperatur inkubiert und 10 µl Enzymprobe erst nach dem Abstoppen mit Trichloressigsäure hinzugegeben. Die Färbereaktion erfolgt analog den anderen Messwerten. Für die Ermittlung der relativen Aktivität wird die höchste gemessene Aktivität auf 100 % gesetzt. Die Ergebnisse sind in Figur 1 dargestellt.

**Temperaturprofil der Phytase Fus5#2:**

| | | | | | | | | | | | |
|---|---|---|---|---|---|---|---|---|---|---|---|
| Temperatur [°C] | 49,4 | 51 | 53,3 | 56,2 | 59,4 | 62,6 | 65,8 | 68,5 | 70,8 | 72,2 | 72,6 |
| Relative Aktivität [%] | 72,3 | 77,4 | 82,2 | 89,6 | 95,5 | 100,0 | 96,5 | 73,1 | 31,7 | 16,9 | 15,9 |

Das Temperaturoptimum der Fus5#2 Phytase liegt bei ca. 63 °C.

### Bestimmung des pH Optimums

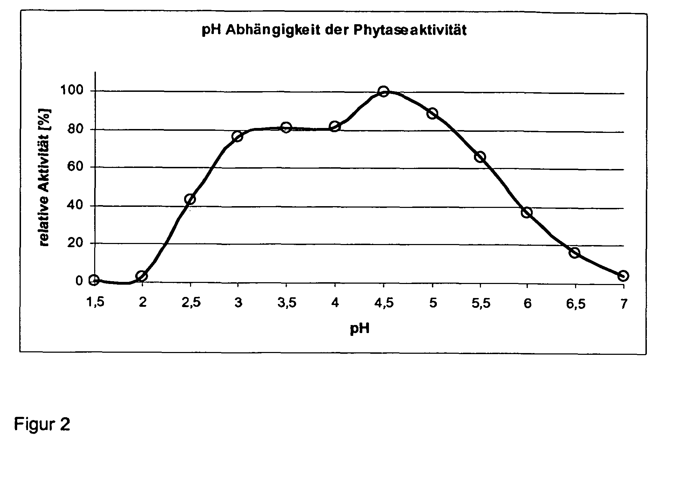

Für die Bestimmung des pH Optimums wird für den Phytaseassay ein modifizierter Reaktionspuffer (100 mM Na-Acetat, 100 mM Glycin, 100 mM Imidazol, 1 mM CaCl₂, 0,01% Tween 20) verwendet, der mit verdünnter Salzsäure auf pH Werte im Bereich von pH 1,5 - 7 eingestellt wird. Für die Ermittlung der relativen Aktivität wird die höchste gemessene Aktivität auf 100 % gesetzt. Die Ergebnisse sind in Figur 2 dargestellt.

**pH Profil der Phytase Fus5#2:**

| | | | | | | | | | | | | |
|---|---|---|---|---|---|---|---|---|---|---|---|---|
| pH Wert | 1,5 | 2 | 2,5 | 3 | 3,5 | 4 | 4,5 | 5 | 5,5 | 6 | 6,5 | 7 |
| relative Aktivität [%] | 0,8 | 2,8 | 43,2 | 76,1 | 81,1 | 81,5 | 100,0 | 88,9 | 66,1 | 37,2 | 15,9 | 4,1 |

Das pH Optimum der Fus5#2 Phytase liegt bei pH 4,5.

### Bestimmung der Thermostabilität (T₅₀)

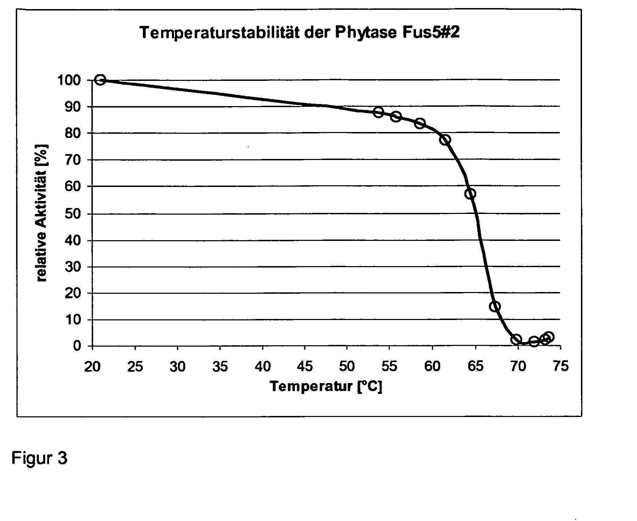

Zur Aufnahme der thermischen Inaktivierungskurve wird die in Reaktionspuffer (250 mM Na-Acetat, 1 mM CaCl₂, 0,01 % Tween 20, pH 5,5) verdünnte Enzymprobe für 20 min auf die jeweilige Temperaturen erhitzt und anschließend auf 4 °C abgekühlt. Eine nicht thermisch behandelte Referenzprobe wird für 20 min bei Raumtemperatur belassen und danach ebenfalls auf 4 °C gekühlt. Im Anschluss an die thermische Vorbehandlung wird die Enzymaktivität der Proben mittels des Phytaseassays bestimmt. Die Aktivität der Referenzprobe wird auf 100 % normiert. Die Thermostabilität der verschiedenen Phytase Varianten wird durch den sogenannten T₅₀ Wert charakterisiert. Der T₅₀ gibt die Temperatur an, bei der nach thermischer Inaktivierung noch 50 % Restaktivität im Vergleich zu einer nicht thermisch behandelten Referenzprobe besteht. Veränderungen in der Thermostabilität zweier Phytase Varianten, ausgedrückt in °C, ergeben sich durch die Differenz der jeweiligen T₅₀ Werte. Die Ergebnisse sind in Figur 3 dargestellt.

**T₅₀ Bestimmung der Phytase Fus5#2:**

| | | | | | | | | | | | |
|---|---|---|---|---|---|---|---|---|---|---|---|
| Temperatur [°C] | 21 | 53,8 | 55,9 | 58,6 | 61,5 | 64,5 | 67,4 | 69,9 | 72 | 73,3 | 73,7 |
| Restaktivität [%] | 100 | 88 | 86 | 83 | 77 | 57 | 15 | 2 | 1 | 2 | 3 |

| | | | | | | | | | | | |
|---|---|---|---|---|---|---|---|---|---|---|---|
| Für die Fus5#2 Phytase ergibt sich hieraus ein T₅₀ -Wert von 65 °C. | | | | | | | | | | | |

### 2. Phytasevarianten von Phytase Fus5#2

Varianten der Phytase wurden durch Mutation der Gensequenz SEQ ID NO: 19 mittels PCR erzeugt. Für eine gezielte Mutagenese wird das "Quickchange Site-directed Mutagenesis Kit" (Stratagene) verwendet. Eine Zufallsmutagenese über den gesamten oder auch nur einen Teil der kodierenden Sequenz aus SEQ ID NO: 19 wird mit Hilfe des "GeneMorph II Random Mutagenesis Kit" (Stratagene) durchgeführt. Die Mutageneserate wird über die Menge der verwendeten Template-DNA auf das gewünschte Maß von 1-5 Mutationen eingestellt. Mehrfachmutationen werden durch die gezielte Kombination von Einzelmutationen oder durch das sequentielle Durchführen mehrerer Mutageneserunden erzeugt.
Auf diese Art erzeugte Varianten des Phytasegens werden analog der ursprünglichen Phytase Fus5#2 in den *E. coli* Expressionsvektor pET22b (Novagen) kloniert und anschließend mit Hilfe des *E. coli* Stammes BL21(DE3) exprimiert.
Vereinzelt werden ausgewählte Phytasevarianten analog zur Ausgangsphytase Fus5#2 mit Hilfe eines entsprechenden Expressionskonstruktes in *Aspergillus niger* exprimiert.

Die erzeugten Phytasevarianten werden in einem hochdurchsatzfähigen Test auf Phytaseaktivität und Temperaturstabilität überprüft. Hierzu werden die nach der Transformation mit dem pET22b basierten Expressionskonstrukt erhaltenen *E. coli* BL21(DE3) Klone, in 96 Loch Mikrotiterplatten in LB Medium (2 % Glucose, 100 mg/L Ampicillin) inkubiert (30 °C, 900 U/min, 2 mm Schüttlerauslenkung). Bei einer OD (600 nm) von ungefähr 0,5 wird mit 1 mM IPTG für 4 h induziert. Im Anschluss wird 10 % (v/v) einer 10x BugBuster Lösung (Novogen) zugegeben und für 15 min bei Raumtemperatur inkubiert. Phytaseaktivität und Restaktivität nach einem 20 minütigen Temperaturstress werden bestimmt. Für Varianten mit erhöhter relativer Restaktivität wird die Thermostabilität (T₅₀) bestimmt. Für einige ausgewählte Phytasevarianten werden zusätzliche charakteristische Parameter (z. B. Temperaturoptimum, spezifische Aktivität, pH Optimum) ermittelt.

### Thermostabilität

Die Steigerung der Thermostabilität der einzelnen Phytasevarianten wird durch ΔT ausgedrückt, wobei sich ΔT die Erhöhung in °C des T₅₀ Wertes im Vergleich zur Phytase Fus5#2 angibt. Die Angabe der Mutation bezieht sich auf das Ausgangsmolekül Fus5#2.

**Tabelle 1: Erhöhung der Thermostabilität der Phytasevarianten mit 1 bis 14 Mutationen gegenüber der synthetischen Phytase Fus5#2 in °C**

| Mutantenummern | Mutation | ΔT [°C] |
|---|---|---|
| Fus5#2 | SEQ ID NO 18 | 0 |
| A-4 | Q349R | 1 |
| A-10 | A84V / A304V | 1 |
| A-66 | H228Y | 1 |
| A-73 | N202S | 1 |
| C-7 | T320L / H413L | 1 |
| C-40 | K234N | 1 |
| X-1 | Q256Y | 1 |
| X-2 | K207E / A209S / N270K | 1 |
| A-164 | A6V | 1 |
| B-16 | K207E | 1 |
| B-378 | H143Y | 1 |
| C-79 | Q109K / D247K | 1 |
| A-11 | Q256H / K402N | 1,5 |
| X-6 | K207E / A209S | 1,5 |
| B-320 | M137L / K207T | 1,5 |
| A-508 | Q349R / H228Y / A304V | 1,5 |
| A-8 | K234I / K251N / H413Q | 2 |
| A-20 | D92E | 2 |
| A-507 | N202S / H228Y | 2 |
| X-3 | D92P | 2,5 |
| A-505 | D92E / N202S | 2,5 |
| A-501 | D92E / K234I / K251N / H413Q | 3 |
| A-407 | A89T / D92A / N270K | 3 |
| A-502 | D92E / Q256H | 3,5 |
| X-4 | A89T / D92A | 3 |
| A-408 | A89T / D92A / K207E / A209S | 3,5 |
| A-415 | A89T / D92A / S261E | 3,5 |
| A-501 | D92E / K234I / K251N / H413Q | 3,5 |
| A-409 | A89T / D92A / S248L / Q256Y | 4 |
| A-503 | D92E / K234I / K251N / Q256H / H413Q | 5 |
| A-406 | A89T / D92A / Q256Y | 5 |
| A-510 | D92E / N202S / K234I / K251N / Q256Y / H413Q / K207E / A209S | 5 |
| A-515 | D92E / N202S / A209S / K234I / Q256Y / H413Q | 5 |
| D-5 | D92E / A142T/ K234I / K251N / Q256H / H413Q | 5,5 |
| D-34 | S1N / S17N/ D92E / K234I / K251N / Q256H / H413Q | 5,5 |
| F-161 | K12N / D92E / K234I / K251N / Q256H / H413Q | 5,5 |
| A-504 | D92E / N202S / K234I / K251N / Q256H / H413Q | 6 |
| D-192 | D92E / S140P / K207I / K234I / K251N / Q256H / H413Q | 6 |
| A-511 | D92E / M137L / N202S / K234I / K251N / Q256H / H413Q | 6 |
| A-514 | D92E / N202S / K234I / K251N / Q256H / K402N H413Q | 6 |
| A-516 | D92E / N202S / K234I / E243K / K251N / Q256H / H413Q | 6 |
| F-41 | D92E / D138N / K234I / K251N / Q256H / H413Q | 6,5 |
| D-207 | D92E / Q149H / K234I / K251N / Q256H / H413Q | 6,5 |
| D-268 | D92E / T156R / K234I / K251N / Q256H / H413Q | 6,5 |
| F-150 | D92E / K234I / K251 N / A255V / Q256H / H413Q | 6,5 |
| I-117 | D92E / N202T / K234I / K251N / Q256H / S373I / E382G / T399I / H413Q | 6,5 |
| A-509 | A89T / D92A / N202S / K234I / K251N / Q256H / H413Q | 6,5 |
| H-107 | D92E / N202S / K234I / K251N / Q256H / H413Q | 7 |
| H-159 | A89T / D92A / N202S / K207E / K234I / K251 N / Q256H | 7 |
| H-456 | A89T / D92A / K207E / K234I / K251N / Q256H / H413Q | 7 |
| A-512 | D92E / H143Y / K234I / K251N / Q256H / H413Q | 7 |
| H-464 | A89T / D92A / G205R / K207E / V208M / K251 N / Q256H | 7,5 |
| A-513 | D92E / H/228Y / K234I / K251N / Q256H / H413Q | 7,5 |
| A-518 | A89T / D92A / H143Y / N202S / K207E / A209S / H228Y / K234I / K251N / Q256H / H413Q | 10 |
| A-521 | A89T / D92N / A142T / H143Y / N202S / K207E / A209S / H228Y / K234I / D247K / K251N / Q256H / F356L / H413Q | 10 |
| A-534 | A89T / D92A / H143Y / T156R / N202S / K207E / A209S / H228Y / K234I / K251N / Q256H / S314G / H413Q | 11 |
| A-519 | A89T / D92A / A142T / H143Y / N202S / K207E / A209S / H228Y / K234I / D247K / K251 N / Q256H / H413Q | 11 |

**Tabelle 2: Erhöhung der spezifischen Aktivität von Phytasevarianten gegenüber der synthetischen Phytase Fus5#2.**

| Mutantenummern | Mutation | Relative spezifische Aktivität [%] |
|---|---|---|
| Fus5#2 | SEQ ID NO 18 | 100 |
| X-5 | D92N | 120 |

## Patentansprüche

1. Eine Phytase, **dadurch gekennzeichnet, dass** sie mindestens 90 % Identität zur Aminosäuresequenz der Seq ID 18 und eine erhöhte Thermostabilität gegenüber den beiden Wildtyp Phytasen aus *Yersinia mollaretii* und *Hafnia sp.* aufweist.

2. Phytase nach Anspruch 1, **dadurch gekennzeichnet, dass** sie mindestens an einer der Positionen ausgewählt aus der Gruppe bestehend aus Position
1, 6, 12, 17, 84, 89, 92, 109, 137, 138, 140, 142, 143, 149, 156, 202, 205, 207, 208, 209, 228, 234, 243, 247, 248, 251, 255, 256, 261, 270, 304, 314, 320, 349, 356, 373, 382, 399, 402 und 413 bezogen auf die Position gemäß Seq ID 18, eine Veränderung der Aminosäure aufweist.

3. Phytase nach Anspruch 2, **dadurch gekennzeichnet, dass** sie mindestens 5 Stück der Veränderungen aufweist.

4. Phytase nach Anspruch 1, **dadurch gekennzeichnet, dass** mindestens eine der Aminosäuren an Position 1, 6, 12, 17, 84, 89, 92, 109, 137, 138, 140, 142, 143, 149, 156, 202, 205, 207, 208, 209, 228, 234, 243, 247, 248, 251, 255, 256, 261, 270, 304, 314, 320, 349, 356, 373, 382, 399, 402 und 413, bezogen auf die Position gemäß Seq ID 18,
durch jeweils eine der Aminosäuren 1 N, D, Q, H; 6 V, I, L, T, S; 12 N, D, Q, H, S; 17 N, D, Q, H; 84 V, I, L, T, S; 89 T, S, V; 92 E, Q, K, R, N, P, A, S, G, H; 109 K, R, E, D; 137 L, I, V; 138 N, Q, H, S; 140 P, A, N; 142 T, S, V; 143 Y, F, W; 149 H, N, S; 156 R, K, E, D; 202 S, T, A, V; 205 R, K, E, D; 207 E, Q, D, R, N, T, S, V, A, I, L, M; 208 M, L, I; 209 S, T; 228 Y, F, W; 234 N, D, Q, H, S, I, V, L, M; 243 K, R, D; 247 K, R, E; 248 L, I, M, V; 251 N, D, Q, H, S, I, V, L, M; 255 V, I, L, T, S; 256 Y, F, W, H, N, S; 261 E, Q, D, K, R, N; 270 K, R, E, D; 304 V, I, L, T, S; 314 G, A; 320 L, I, M, V; 349 R, K, E, D; 356 L, I, M, V; 373 I, V, L, M; 382 G, A; 399 I, V, L, M; 402 N, D, Q, H, S; 413 L, I, M, V, Q, E, N, K, R ausgetauscht ist.

5. Phytase nach Anspruch 1, **dadurch gekennzeichnet, dass** sie mindestens eine der Veränderungen ausgewählt aus der Gruppe bestehend aus S1N; A6V; K12N; S17N; A84V; A89T; D92E; D92P; D92A D92N; Q109K; M137L; D138N; S140P; A142T; H143Y; Q149H; T156R; N202S; N202T; G205R; K207E; K207T; K2071; V208M; A209S; H228Y; K234N; K234I; E243K; D247K; S248L; K251 N; K251I; A255V; Q256Y; Q256H; S261 E; N270K; A304V; S314G; T320L; Q349R; F356L; S373I; E382G; T399I; K402N; H413L und H413Q
gegenüber der Aminosäuresequenz der Seq ID 18 aufweist.

6. Phytase nach Anspruch 5, **dadurch gekennzeichnet, dass** sie mindestens 5 Stück der Veränderungen aufweist.

7. Phytase nach Anspruch 1, **dadurch gekennzeichnet, dass** sie mindestens eine der Veränderungen ausgewählt aus der Gruppe bestehend aus
[A89T / D92A / H143Y / N202S / K207E / A209S / H228Y / K234I / K251 N / Q256H / H413Q],
[A89T / D92N / A142T / H143Y / N202S / K207E / A209S / H228Y / K234I / D247K / K251 N / Q256H / F356L / H413Q],
[A89T / D92A / H143Y / T156R / N202S / K207E / A209S / H228Y / K234I / K251 N / Q256H / S314G / H413Q] und
[A89T / D92A / A142T / H143Y / N202S / K207E / A209S / H228Y / K234I / D247K / K251 N / Q256H / H413Q]
aufweist.

8. Eine Phytase nach einem der vorhergehenden Ansprüche, **dadurch gekennzeichnet, dass** sie mindestens einen konservativen Aminosäuereaustausch an mindestens einer Position gegenüber einer der Phytasen gemäss einem der Ansprüche 5 bis 7 aufweist.

9. Phytase nach einem der vorhergehenden Ansprüche, **dadurch gekennzeichnet, dass** es sich um eine isolierte Phytase handelt.

10. Phytase nach einem der vorhergehenden Ansprüche, **dadurch gekennzeichnet, dass** sie eine erhöhte spezifische Aktivität gegenüber den beiden Wildtyp Phytasen aus *Yersinia mollaretii* und *Hafnia sp.* aufweist.

11. Isolierte Nukleinsäuresequenz codierend eine Phytase, **dadurch gekennzeichnet, dass** sie eine der Phytasen gemäß Anspruch 1 bis 10 codiert.

12. Isolierte Nukleinsäuresequenz codierend eine Phytase, **dadurch gekennzeichnet, dass** sie eine erhöhte Thermostabilität gegenüber den beiden Wildtyp Phytasen aus *Yersinia mollaretii* und *Hafnia sp.* aufweist
a) mindestens 90 % Identität zur Nukleinsäure Sequenz der Seq ID 19 aufweist, oder
b) mit dem komplementären Strang einer der Sequenzen aus a) unter hoch stringenten Bedingungen hybridisiert.

13. Rekombinanter Expressionsvektor enthaltend eine Nukleinsäuresequenz gemäß Anspruch 11 oder 12.

14. Rekombinante Wirtszelle enthaltend eine Nukleinsäuresequenz gemäß Anspruch 11 oder 12 oder den Vektor gemäß Anspruch 13.

15. Rekombinanter Produktionsorganismus enthaltend eine Nukleinsäuresequenz gemäß Anspruch 11 oder 12 oder den Vektor gemäß Anspruch 13.

16. Tierfuttermittelzusatz enthaltend mindestens eine der Phytasen gemäß einem der Ansprüche 1 bis 10 sowie weitere Futtermittelzusatzstoffe.

17. Tierfuttermittel enthaltend mindestens eine der Phytasen gemäß einem der Ansprüche 1 bis 10.

18. Verwendung einer Phytase gemäß einem der Ansprüche 1 bis 10 oder des Tierfuttermittelzusatzes gemäß Anspruch 16 in einem Tierfuttermittel.

19. Verwendung einer Phytase gemäß einem der Ansprüche 1 bis 10, des Tierfuttermittelzusatzes gemäß Anspruch 16 oder des Tierfuttermittels gemäss Anspruch 17 zur Reduktion des Phosphatgehalts in der Gülle von Nutztieren.

## Claims

1. A phytase which has at least 90% identity with the amino acid sequence of SEQ ID 18 and an elevated thermal stability with respect to the two wild type phytases from *Yersinia mollaretìì* and *Hafnia sp.*

2. The phytase according to claim 1, wherein it has a change of the amino acid at at least one of the positions selected from the group consisting of position
1, 6, 12, 17, 84, 89, 92, 109, 137, 138, 140, 142, 143, 149, 156, 202, 205, 207, 208, 209, 228, 234, 243, 247, 248, 251, 255, 256, 261, 270, 304, 314, 320, 349, 356, 373, 382, 399, 402 and 413, based on the position according to SEQ ID 18.

3. The phytase according to claim 2, wherein it has at least 5 of the changes.

4. The phytase according to claim 1, wherein at least one of the amino acids at position 1, 6, 12, 17, 84, 89, 92, 109, 137, 138, 140, 142, 143, 149, 156, 202, 205, 207, 208, 209, 228, 234, 243, 247, 248, 251, 255, 256, 261, 270, 304, 314, 320, 349, 356, 373, 382, 399, 402 and 413, based on the position according to SEQ ID 18,
is replaced by in each case one of the amino acids 1 N, D, Q, H; 6 V, I, L, T, S; 12 N, D, Q, H, S; 17 N, D, Q, H; 84 V, I, L, T, S; 89 T, S, V; 92 E, Q, K, R, N, P, A, S, G, H; 109 K, R, E, D; 137 L, I, V; 138 N, Q, H, S; 140 P, A, N; 142 T, S, V; 143 Y, F, W; 149 H, N, S; 156 R, K, E, D; 202 S, T, A, V; 205 R, K, E, D; 207 E, Q, D, R, N, T, S, V, A, I, L, M; 208 M, L, I; 209 S, T; 228 Y, F, W; 234 N, D, Q, H, S, I, V, L, M; 243 K, R, D; 247 K, R, E; 248 L, I, M, V; 251 N, D, Q, H, S, I, V, L, M; 255 V, I, L, T, S; 256 Y, F, W, H, N, S; 261 E, Q, D, K, R, N; 270 K, R, E, D; 304 V, I, L, T, S; 314 G, A; 320 L, I, M, V; 349 R, K, E, D; 356 L, I, M, V; 373 I, V, L, M; 382 G, A; 399 I, V, L, M; 402 N, D, Q, H, S; 413 L, I, M, V, Q, E, N, K, R.

5. The phytase according to claim 1, wherein it has at least one of the changes selected from the group consisting of
S1N; A6V; K12N; S17N; A84V; A89T; D92E; D92P; D92A; D92N; Q109K; M137L; D138N; S140P; A142T; H143Y; Q149H; T156R; N202S; N202T; G205R; K207E; K207T; K207I; V208M; A209S; H228Y; K234N; K234I; E243K; D247K; S248L; K251N; K251I; A255V; Q256Y; Q256H; S261E; N270K; A304V; S314G; T320L; Q349R; F356L; S373I; E382G; T399I; K402N; H413L and H413Q
with respect to the amino acid sequence of SEQ ID 18.

6. The phytase according to claim 5, wherein it has at least 5 of the changes.

7. The phytase according to claim 1, wherein it has at least one of the changes selected from the group consisting of
[A89T / D92A / H143Y / N202S / K207E / A209S / H228Y / K234I / K251N / Q256H / H413Q],
[A89T / D92N / A142T / H143Y / N202S / K207E / A209S / H228Y / K234I / D247K / K251N / Q256H / F356L / H413Q],
[A89T / D92A / H143Y / T156R / N202S / K207E / A209S / H228Y / K234I / K251N / Q256H / S314G / H413Q] and
[A89T / D92A / A142T / H143Y / N202S / K207E / A209S / H228Y / K234I / D247K / K251N / Q256H / H413Q].

8. A phytase according to any one of the preceding claims, wherein it has at least one conservative amino acid replacement at at least one position with respect to one of the phytases according to any one of claims 5 to 7.

9. The phytase according to any one of the preceding claims, wherein it is an isolated phytase.

10. The phytase according to any one of the preceding claims, wherein it has an elevated specific activity with respect to the two wild type phytases from *Yersinia mollaretii* and *Hafnia sp.*

11. An isolated nucleic acid sequence encoding a phytase which encodes one of the phytases according to claims 1 to 10.

12. An isolated nucleic acid sequence encoding a phytase which has an elevated thermal stability with respect to the two wild type phytases from *Yersinia mollaretii* and *Hafnia sp.*
a) has at least 90% identity with the nucleic acid sequence of SEQ ID 19, or
b) hybridizes with the complementary strand of one of the sequences of a) under highly stringent conditions.

13. A recombinant expression vector comprising a nucleic acid sequence according to claim 11 or 12.

14. A recombinant host cell comprising a nucleic acid sequence according to claim 11 or 12 or the vector according to claim 13.

15. A recombinant production organism comprising a nucleic acid sequence according to claim 11 or 12 or the vector according to claim 13.

16. An animal feed additive comprising at least one of the phytases according to any one of claims 1 to 10 and also further feed additives.

17. An animal feed comprising at least one of the phytases according to any one of claims 1 to 10.

18. The use of a phytase according to any one of claims 1 to 10 or the animal feed additive according to claim 16 in an animal feed.

19. The use of a phytase according to any one of claims 1 to 10, the animal feed additive according to claim 16, or the animal feed according to claim 17 for reducing the phosphate content in the manure of farm animals.

## Revendications

1. Phytase, **caractérisée en ce qu'**elle présente une identité d'au moins 90 % avec la séquence d'acides aminés Seq ID 18 et une thermostabilité accrue par comparaison avec les deux phytases de type sauvage de *Yersinia mollaretii* et de *Hafnia sp.*

2. Phytase selon la revendication 1, **caractérisée en ce qu'**elle présente une modification de l'acide aminé au moins sur l'une des positions choisie dans le groupe consistant en les positions 1, 6, 12, 17, 84, 89, 92, 109, 137, 138, 140, 142, 143, 149, 156, 202, 205, 207, 208, 209, 228, 234, 243, 247, 248, 251, 255, 256, 261, 270, 304, 314, 320, 349, 356, 373, 382, 399, 402 et 413, par rapport à la position selon Seq ID 18.

3. Phytase selon la revendication 2, **caractérisée en ce qu'**elle comprend au moins 5 éléments des modifications.

4. Phytase selon la revendication 1, **caractérisée en ce qu'**au moins l'un des acides aminés en positions 1, 6, 12, 17, 84, 89, 92, 109, 137, 138, 140, 142, 143, 149, 156, 202, 205, 207, 208, 209, 228, 234, 243, 247, 248, 251, 255, 256, 261, 270, 304, 314, 320, 349, 356, 373, 382, 399, 402 et 413 est, par rapport à la position selon Seq ID 18, remplacé par chacun des acides aminés suivants : 1 N, D, Q, H ; 6 V, I, L, T, S ; 12 N, D, Q, H, S ; 17 N, D, Q, H ; 84 V, I, L, T, S ; 89 T, S, V ; 92 E, Q, K, R, N, P, A, S, G, H ; 109 K, R, E, D ; 137 L, I, V ; 138 N, Q, H, S ; 140 P, A, N ; 142 T, S, V ; 143 Y, F, W ; 149 H, N, S ; 156 R, K, E, D ; 202 S, T, A, V ; 205 R, K, E, D ; 207 E, Q, D, R, N, T, S, V, A, I, L, M ; 208 M, L, I ; 209 S, T ; 228 Y, F, W ; 234 N, D, Q, H, S, I, V, L, M ; 243 K, R, D ; 247 K, R, E ; 248 L, I, M, V ; 251 N, D, Q, H, S, I, V, L, M ; 255 V, I, L, T, S ; 256 Y, F, W, H, N, S ; 261 E, Q, D, K, R, N ; 270 K, R, E, D ; 304 V, I, L, T, S ; 314 G, A ; 320 L, I, M, V ; 349 R, K, E, D ; 356 L, I, M, V ; 373 I, V, L, M ; 382 G, A ; 399 I, V, L, M ; 402 N, D, Q, H, S ; 413 L, I, M, V, Q, E, N, K, R.

5. Phytase selon la revendication 1, **caractérisée en ce qu'**elle comprend au moins l'une des modifications choisies dans le groupe consistant en S1N ; A6V ; K12N ; S17N ; A84V ; A89T ; D92E ; D92P ; D92A ; D92N ; Q109K ; M137L ; D138N ; S140P ; A142T ; H143Y ; Q149H ; T156R ; N202S ; N202T ; G205R ; K207E ; K207T ; K207I ; V208M ; A209S ; H228Y ; K234N ; K234I ; E243K ; D247K ; S248L ; K251N ; K251I ; A255V ; Q256Y ; Q256H ; S261E ; N270K ; A304V ; S314G ; T320L ; Q349R ; F356L ; S373I ; E382G ; T399I ; K402N ; H413L et H413Q, par rapport à la séquence d'acides aminés Seq ID 18.

6. Phytase selon la revendication 5, **caractérisée en ce qu'**elle comprend au moins 5 éléments des modifications.

7. Phytase selon la revendication 1, **caractérisée en ce qu'**elle comprend au moins l'une des modifications choisies dans le groupe consistant en
[A89T/D92A/H143Y/N202S/K207E/A209S/H228Y/K234I/K251N/ Q256H/H413Q],
[A89T/D92N/A142T/H143Y/N202S/K207E/A209S/H228Y/K234I/ D247K/K251N/Q256H/F356L/H413Q],
[A89T/D92A/H143Y/T156R/N202S/K207E/A209S/H228Y/K234I/ K251N/Q256H/S314G/H413Q] et
[A89T/D92A/A142T/H143Y/N202S/K207E/A209S/H228Y/K234I/ D247K/K251N/Q256H/H413Q.

8. Phytase selon l'une des revendications précédentes, **caractérisée en ce qu'**elle comprend au moins un échange conservatif d'acides aminés sur au moins une position, par rapport à l'une des phytases selon l'une des revendications 5 à 7.

9. Phytase selon l'une des revendications précédentes, **caractérisée en ce qu'**il s'agit d'une phytase isolée.

10. Phytase selon l'une des revendications précédentes, **caractérisée en ce qu'**elle présente une activité spécifique accrue par comparaison avec les deux phytases de type sauvage de *Yersinia mollaretii* et de *Hafnia sp.*

11. Séquence d'acide nucléique isolée codant pour une phytase, **caractérisée en ce qu'**elle code pour l'une des phytases selon les revendications 1 à 10.

12. Séquence d'acide nucléique isolée codant pour une phytase, **caractérisée en ce qu'**elle présente une thermostabilité accrue par comparaison avec les deux phytases de type sauvage de *Yersinia mollaretii* et de *Hafnia sp.,*
a) qu'elle présente une identité d'au moins 90 % avec la séquence d'acide nucléique Seq ID 19, ou
b) qu'elle s'hybride au brin complémentaire de l'une des séquences de a) dans des conditions hautement stringentes.

13. Vecteur d'expression recombinant contenant une séquence d'acide nucléique selon la revendication 11 ou 12.

14. Cellule hôte recombinante contenant une séquence d'acide nucléique selon la revendication 11 ou 12 ou le vecteur selon la revendication 13.

15. Organisme de production recombinant contenant une séquence d'acide nucléique selon la revendication 11 ou 12 ou le vecteur selon la revendication 13.

16. Additif pour produits pour l'alimentation animale contenant au moins l'une des phytases selon l'une des revendications 1 à 10, ainsi que d'autres additifs pour produits pour l'alimentation animale.

17. Produit pour l'alimentation animale contenant au moins l'une des phytases selon l'une des revendications 1 à 10.

18. Utilisation d'une phytase selon l'une des revendications 1 à 10 ou de l'additif pour produits pour l'alimentation animale selon la revendication 16 dans un produit pour l'alimentation animale.

19. Utilisation d'une phytase selon l'une des revendications 1 à 10, de l'additif pour produits pour l'alimentation animale selon la revendication 16 ou du produit pour l'alimentation animale selon la revendication 17 pour réduire la teneur en phosphates du lisier du bétail.


### SEQUENCE LISTING

<110> BASF SE
<120> Synthetische Phytasevarianten
<130> PF 62585
<140>
   <141> 2009-10-22
<160> 23
<170> PatentIn version 3.4
<210> 1
   <211> 17
   <212> DNA
   <213> Artificial Sequence
<220>
   <223> Primer
<220>
   <221> misc_feature
   <222> (6)..(6)
   <223> n is a, c, g, or t
<220>
   <221> misc_feature
   <222> (9)..(9)
   <223> n is a, c, g, or t
<400> 1
   gayccnytnt tycaycc 17
<210> 2
   <211> 17
   <212> DNA
   <213> Artificial Sequence
<220>
   <223> Primer
<220>
   <221> misc_feature
   <222> (3)..(3)
   <223> n is a, c, g, or t
<220>
   <221> misc_feature
   <222> (12)..(12)
   <223> n is a, c, g, or t
<400> 2
   ggngtrttrt cnggytg 17
<210> 3
   <211> 17
   <212> DNA
   <213> Artificial Sequence
<220>
   <223> Primer
<220>
   <221> misc_feature
   <222> (9)..(9)
   <223> n is a, c, g, or t
<400> 3
   gcdatrttng trtcrtg 17
<210> 4
   <211> 16
   <212> DNA
   <213> Artificial Sequence
<220>
   <223> Primer
<220>
   <221> misc_feature
   <222> (5)..(5)
   <223> n is a, c, g, or t
<220>
   <221> misc_feature
   <222> (10)..(10)
   <223> n is a, c, g, or t
<220>
   <221> misc_feature
   <222> (13)..(13)
   <223> n is a, c, g, or t
<400> 4 16
   wcagntgwtn gtnctg 16
<210> 5
   <211> 26
   <212> DNA
   <213> Artificial Sequence
<220>
   <223> Primer
<400> 5
   cttcgagagc cactttatta ccgtcg 26
<210> 6
   <211> 25
   <212> DNA
   <213> Artificial Sequence
<220>
   <223> Primer
<400> 6
   ccaatgttgt gctgctgaca atagg 25
<210> 7
   <211> 25
   <212> DNA
   <213> Artificial Sequence
<220>
   <223> Primer
<400> 7
   ccgaactcat cagcgctaaa gatgc 25
<210> 8
   <211> 16
   <212> DNA
   <213> Artificial Sequence
<220>
   <223> Primer
<220>
   <221> misc_feature
   <222> (8)..(8)
   <223> n is a, c, g, or t
<400> 8
   cawcgwcnga sasgaa 16
<210> 9
   <211> 26
   <212> DNA
   <213> Artificial Sequence
<220>
   <223> Primer
<400> 9
   cgcagtttga cttgatgtcg cgcacg 26
<210> 10
   <211> 26
   <212> DNA
   <213> Artificial Sequence
<220>
   <223> Primer
<400> 10
   gtcgcgcacg ccctatatcg ccaagc 26
<210> 11
   <211> 25
   <212> DNA
   <213> Artificial Sequence
<220>
   <223> Primer
<400> 11
   ctgcaaacca tcgcacacgc actgg 25
<210> 12
   <211> 1341
   <212> DNA
   <213> Hafnia sp.
<220>
   <221> sig_peptide
   <222> (1)..(99)
<400> 12
   atgacaatct ctctgtttaa ccgtaataaa cccgctattg cacagcgtat tttatgtcct     60
   ctgatcgtgg ctttattctc aggtttaccg gcatacgcca gtgataccgc ccctgctggg    120
   ttccagttgg aaaaggttgt tatcctaagc agacatggcg tacgcgcgcc aaccaaaatg    180
   acacaaacga tgcgcaacgt cacacctcac cagtggcctg aatggccggt aaaactcggc    240
   tatatcacgc cccgcggtga acatctgatt agcctgatgg gcggttttta tcgagagcgc    300
   tttcagcaac aaggcttatt acctaaggat aactgtccta caccagatgc cgtgtatgtt    360
   tgggcagacg tcgatcaacg cacacgtaaa accggcgagg ccttcttagc gggtcttgct    420
   ccccagtgtg atttagcgat ccaccatcag caaaacattc agcaggccga tccgctgttc    480
   catcctgtga aagccggtat ctgttcgatg gataaatcac aggcacacgc cgccgttgaa    540
   aagcaggcag gcacaccgat tgagacgctc aatcaacgct atcaagcatc tttagcgctg    600
   atgagttcgg tactcgattt tccaaaatcc ccctattgtc agcagcacaa cattggcaaa    660
   ctctgcgatt tttcacaggc gatgcctagc aggctggcga taaatgacga cggtaataaa    720
   gtggctctcg aaggtgccgt gggactttca tcgacgttgg ctgaaatttt cctgctggaa    780
   cacgctcagg gaatgcctaa agtggcttgg gggaatattc acactgagca gcaatgggac    840
   tctctgttaa aattgcataa tgcgcagttt gacttgatgt cgcgcacgcc ctatatcgcc    900
   aagcataacg gtactccact gctgcaaacc atcgcacacg cactgggttc caatatcgcg    960
   agtcgcccac tgccggatat ttcgccagac aataagatcc tgtttattgc cggtcacgac   1020
   accaatattg ccaatatttc tggcatgcta gggatgacat ggacacttcc gggacagcca   1080
   gataacacgc ctccgggcgg ggctttagtg tttgaacgtt gggtagataa cgcgggggaa   1140
   ccgtatgtta gcgtgaatat ggtgtatcaa acactggcac agttgcacga ccagacgccg   1200
   ctaacgttgc agcatcctgc gggcagcgta cgactaaaca taccgggttg cagcgatcaa   1260
   acgcccgatg gctattgccc gctctccacc ttcagccgtt tagtcaacca cagcgttgag   1320
   cctgcgtgcc agcttcctta a                                             1341
<210> 13
   <211> 446
   <212> PRT
   <213> Hafnia sp.
<220>
   <221> SIGNAL
   <222> (1)..(33)
<400> 13
   Met Thr Ile Ser Leu Phe Asn Arg Asn Lys Pro Ala Ile Ala Gln Arg
   1               5                   10                  15
   Ile Leu Cys Pro Leu Ile Val Ala Leu Phe Ser Gly Leu Pro Ala Tyr
               20                  25                  30
   Ala Ser Asp Thr Ala Pro Ala Gly Phe Gln Leu Glu Lys Val Val Ile
           35                  40                  45
   Leu Ser Arg His Gly Val Arg Ala Pro Thr Lys Met Thr Gln Thr Met
       50                  55                  60
   Arg Asn Val Thr Pro His Gln Trp Pro Glu Trp Pro Val Lys Leu Gly
   65                  70                  75                  80
   Tyr Ile Thr Pro Arg Gly Glu His Leu Ile Ser Leu Met Gly Gly Phe
                   85                  90                  95
   Tyr Arg Glu Arg Phe Gln Gln Gln Gly Leu Leu Pro Lys Asp Asn Cys
               100                 105                 110
   Pro Thr Pro Asp Ala Val Tyr Val Trp Ala Asp Val Asp Gln Arg Thr
           115                 120                 125
   Arg Lys Thr Gly Glu Ala Phe Leu Ala Gly Leu Ala Pro Gln Cys Asp
       130                 135                 140
   Leu Ala Ile His His Gln Gln Asn Ile Gln Gln Ala Asp Pro Leu Phe
   145                 150                 155                 160
   His Pro Val Lys Ala Gly Ile Cys Ser Met Asp Lys Ser Gln Ala His
                   165                 170                 175
   Ala Ala Val Glu Lys Gln Ala Gly Thr Pro Ile Glu Thr Leu Asn Gln
               180                 185                 190
   Arg Tyr Gln Ala Ser Leu Ala Leu Met Ser Ser Val Leu Asp Phe Pro
           195                 200                 205
   Lys Ser Pro Tyr Cys Gln Gln His Asn Ile Gly Lys Leu Cys Asp Phe
       210                 215                 220
   Ser Gln Ala Met Pro Ser Arg Leu Ala Ile Asn Asp Asp Gly Asn Lys
   225                 230                 235                 240
   Val Ala Leu Glu Gly Ala Val Gly Leu Ser Ser Thr Leu Ala Glu Ile
                   245                 250                 255
   Phe Leu Leu Glu His Ala Gln Gly Met Pro Lys Val Ala Trp Gly Asn
               260                 265                 270
   Ile His Thr Glu Gln Gln Trp Asp Ser Leu Leu Lys Leu His Asn Ala
           275                 280                 285
   Gln Phe Asp Leu Met Ser Arg Thr Pro Tyr Ile Ala Lys His Asn Gly
       290                 295                 300
   Thr Pro Leu Leu Gln Thr Ile Ala His Ala Leu Gly Ser Asn Ile Ala
   305                 310                 315                 320
   Ser Arg Pro Leu Pro Asp Ile Ser Pro Asp Asn Lys Ile Leu Phe Ile
                   325                 330                 335
   Ala Gly His Asp Thr Asn Ile Ala Asn Ile Ser Gly Met Leu Gly Met
               340                 345                 350
   Thr Trp Thr Leu Pro Gly Gln Pro Asp Asn Thr Pro Pro Gly Gly Ala
           355                 360                 365
   Leu Val Phe Glu Arg Trp Val Asp Asn Ala Gly Lys Pro Tyr Val Ser
       370                 375                 380
   Val Asn Met Val Tyr Gln Thr Leu Ala Gln Leu His Asp Gln Thr Pro
   385                 390                 395                 400
   Leu Thr Leu Gln His Pro Ala Gly Ser Val Arg Leu Asn Ile Pro Gly
                   405                 410                 415
   Cys Ser Asp Gln Thr Pro Asp Gly Tyr Cys Pro Leu Ser Thr Phe Ser
               420                 425                 430
   Arg Leu Val Asn His Ser Val Glu Pro Ala Cys Gln Leu Pro
           435                 440                 445
<210> 14
   <211> 1074
   <212> DNA
   <213> Hafnia sp.
<400> 14
   atgacaatct ctctgtttaa ccgtaataaa cccgctattg cacagcgtat tttatgtcct      60
   ctgatcgtgg ctttattctc aggtttaccg gcatacgcca gtgataccgc ccctgctggg     120
   ttccagttgg aaaaggttgt tatcctaagc agacatggcg tacgcgcgcc aaccaaaatg     180
   acacaaacga tgcgcaacgt cacacctcac cagtggcctg aatggccggt aaaactcggc     240
   tatatcacgc cccgcggtga acatctgatt agcctgatgg gcggttttta tcgagagcgc     300
   tttcagcaac aaggcttatt acctaaggat aactgtccta caccagatgc cgtgtatgtt     360
   tgggcagacg tcgatcaacg cacacgtaaa accggcgagg ccttcttagc gggtcttgct     420
   ccccagtgtg atttagcgat ccaccatcag caaaacattc agcaggccga tccgctgttc     480
   catcctgtga aagccggtat ctgttcgatg gataaatcac aggcacacgc cgccgttgaa     540
   aagcaggcag gcacaccgat tgagacgctc aatcaacgct atcaagcatc tttagcgctg     600
   atgagttcgg tactcgattt tccaaaatcc ccctattgtc agcagcacaa cattggcaaa     660
   ctctgcgatt tttcacaggc gatgcctagc aggctggcga taaatgacga cggtaataaa     720
   gtggctctcg aaggtgccgt gggactttca tcgacgttgg ctgaaatttt cctgctggaa     780
   cacgctcagg gaatgcctaa agtggcttgg gggaatattc acactgagca gcaatgggac     840
   tctctgttaa aattgcataa tgcgcagttt gacttgatgt cgcgcacgcc ctatatcgcc     900
   aagcataacg gtactccact gctgcaaacc atcgcacacg cactgggttc caatatcgcg     960
   agtcgcccac tgccggatat ttcgccagac aataagatcc tgtttattgc cggtcacgac    1020
   accaatattg ccaatatttc tggcatgcta gggatgacat ggacacttcc ggga          1074
<210> 15
   <211> 270
   <212> DNA
   <213> Yersinia mollaretii
<400> 15
   cagcccgata acaccccgcc gggtgggggg ctggtgtttg aactatggca gaatccagat      60
   aaccatcagc aatatgtcgc agttaagatg ttctatcaaa caatggatca gttacgaaat     120
   agtgaaaagt tagacctgaa aagtcatcca gccggtattg ttcccattga gatcgaaggt     180
   tgtgagaaca tcggtacaga caaactttgc cagcttgata ccttccaaaa gagagtggct     240
   caggtgattg aacctgcatg ccatatttaa                                      270
<210> 16
   <211> 1344
   <212> DNA
   <213> Artificial Sequence
<220>
   <223> synthetic construct
<220>
   <221> sig_peptide
   <222> (1)..(99)
<400> 16
   atgacaatct ctctgtttaa ccgtaataaa cccgctattg cacagcgtat tttatgtcct      60
   ctgatcgtgg ctttattctc aggtttaccg gcatacgcca gtgataccgc ccctgctggg     120
   ttccagttgg aaaaggttgt tatcctaagc agacatggcg tacgcgcgcc aaccaaaatg     180
   acacaaacga tgcgcaacgt cacacctcac cagtggcctg aatggccggt aaaactcggc     240
   tatatcacgc cccgcggtga acatctgatt agcctgatgg gcggttttta tcgagagcgc    300
   tttcagcaac aaggcttatt acctaaggat aactgtccta caccagatgc cgtgtatgtt     360
   tgggcagacg tcgatcaacg cacacgtaaa accggcgagg ccttcttagc gggtcttgct     420
   ccccagtgtg atttagcgat ccaccatcag caaaacattc agcaggccga tccgctgttc     480
   catcctgtga aagccggtat ctgttcgatg gataaatcac aggcacacgc cgccgttgaa     540
   aagcaggcag gcacaccgat tgagacgctc aatcaacgct atcaagcatc tttagcgctg     600
   atgagttcgg tactcgattt tccaaaatcc ccctattgtc agcagcacaa cattggcaaa     660
   ctctgcgatt tttcacaggc gatgcctagc aggctggcga taaatgacga cggtaataaa     720
   gtggctctcg aaggtgccgt gggactttca tcgacgttgg ctgaaatttt cctgctggaa     780
   cacgctcagg gaatgcctaa agtggcttgg gggaatattc acactgagca gcaatgggac     840
   tctctgttaa aattgcataa tgcgcagttt gacttgatgt cgcgcacgcc ctatatcgcc     900
   aagcataacg gtactccact gctgcaaacc atcgcacacg cactgggttc caatatcgcg     960
   agtcgcccac tgccggatat ttcgccagac aataagatcc tgtttattgc cggtcacgac    1020
   accaatattg ccaatatttc tggcatgcta gggatgacat ggacacttcc gggacagccc    1080
   gataacaccc cgccgggtgg ggggctggtg tttgaactat ggcagaatcc agataaccat    1140
   cagcaatatg tcgcagttaa gatgttctat caaacaatgg atcagttacg aaatagtgaa    1200
   aagttagacc tgaaaagtca tccagccggt attgttccca ttgagatcga aggttgtgag    1260
   aacatcggta cagacaaact ttgccagctt gataccttcc aaaagagagt ggctcaggtg    1320
   attgaacctg catgccatat ttaa                                           1344
<210> 17
   <211> 447
   <212> PRT
   <213> Artificial Sequence
<220>
   <223> synthetic construct
<220>
   <221> SIGNAL
   <222> (1)..(33)
<400> 17
   Met Thr Ile Ser Leu Phe Asn Arg Asn Lys Pro Ala Ile Ala Gln Arg
   1               5                   10                  15
   Ile Leu Cys Pro Leu Ile Val Ala Leu Phe Ser Gly Leu Pro Ala Tyr
               20                  25                  30
   Ala Ser Asp Thr Ala Pro Ala Gly Phe Gln Leu Glu Lys Val Val Ile
           35                  40                  45
   Leu Ser Arg His Gly Val Arg Ala Pro Thr Lys Met Thr Gln Thr Met
       50                  55                  60
   Arg Asn Val Thr Pro His Gln Trp Pro Glu Trp Pro Val Lys Leu Gly
   65                  70                  75                  80
   Tyr Ile Thr Pro Arg Gly Glu His Leu Ile Ser Leu Met Gly Gly Phe
                   85                  90                  95
   Tyr Arg Glu Arg Phe Gln Gln Gln Gly Leu Leu Pro Lys Asp Asn Cys
               100                 105                 110
   Pro Thr Pro Asp Ala Val Tyr Val Trp Ala Asp Val Asp Gln Arg Thr
           115                 120                 125
   Arg Lys Thr Gly Glu Ala Phe Leu Ala Gly Leu Ala Pro Gln Cys Asp
       130                 135                 140
   Leu Ala Ile His His Gln Gln Asn Ile Gln Gln Ala Asp Pro Leu Phe
   145                 150                 155                 160
   His Pro Val Lys Ala Gly Ile Cys Ser Met Asp Lys Ser Gln Ala His
                   165                 170                 175
   Ala Ala Val Glu Lys Gln Ala Gly Thr Pro Ile Glu Thr Leu Asn Gln
               180                 185                 190
   Arg Tyr Gln Ala Ser Leu Ala Leu Met Ser Ser Val Leu Asp Phe Pro
           195                 200                 205
   Lys Ser Pro Tyr Cys Gln Gln His Asn Ile Gly Lys Leu Cys Asp Phe
       210                 215                 220
   Ser Gln Ala Met Pro Ser Arg Leu Ala Ile Asn Asp Asp Gly Asn Lys
   225                 230                 235                 240
   Val Ala Leu Glu Gly Ala Val Gly Leu Ser Ser Thr Leu Ala Glu Ile
                   245                 250                 255
   Phe Leu Leu Glu His Ala Gln Gly Met Pro Lys Val Ala Trp Gly Asn
               260                 265                 270
   Ile His Thr Glu Gln Gln Trp Asp Ser Leu Leu Lys Leu His Asn Ala
           275                 280                 285
   Gln Phe Asp Leu Met Ser Arg Thr Pro Tyr Ile Ala Lys His Asn Gly
   290 295 300
   Thr Pro Leu Leu Gln Thr Ile Ala His Ala Leu Gly Ser Asn Ile Ala
   305 310 315 320
   Ser Arg Pro Leu Pro Asp Ile Ser Pro Asp Asn Lys Ile Leu Phe Ile
   325 330 335
   Ala Gly His Asp Thr Asn Ile Ala Asn Ile Ser Gly Met Leu Gly Met
   340 345 350
   Thr Trp Thr Leu Pro Gly Gln Pro Asp Asn Thr Pro Pro Gly Gly Gly
   355 360 365
   Leu Val Phe Glu Leu Trp Gln Asn Pro Asp Asn His Gln Gln Tyr Val
   370 375 380
   Ala Val Lys Met Phe Tyr Gln Thr Met Asp Gln Leu Arg Asn Ser Glu
   385 390 395 400
   Lys Leu Asp Leu Lys Ser His Pro Ala Gly Ile Val Pro Ile Glu Ile
   405 410 415
   Glu Gly Cys Glu Asn Ile Gly Thr Asp Lys Leu Cys Gln Leu Asp Thr
   420 425 430
   Phe Gln Lys Arg Val Ala Gln Val Ile Glu Pro Ala Cys His Ile
   435 440 445
<210> 18
   <211> 414
   <212> PRT
   <213> Artificial Sequence
<220>
   <223> chimeric protein
<400> 18
   Ser Asp Thr Ala Pro Ala Gly Phe Gln Leu Glu Lys Val Val Ile Leu
   1               5                   10                  15
   Ser Arg His Gly Val Arg Ala Pro Thr Lys Met Thr Gln Thr Met Arg
               20                  25                  30
   Asn Val Thr Pro His Gln Trp Pro Glu Trp Pro Val Lys Leu Gly Tyr
           35                  40                  45
   Ile Thr Pro Arg Gly Glu His Leu Ile Ser Leu Met Gly Gly Phe Tyr
   50 55 60
   Arg Glu Arg Phe Gln Gln Gln Gly Leu Leu Pro Lys Asp Asn Cys Pro
   65 70 75 80
   Thr Pro Asp Ala Val Tyr Val Trp Ala Asp Val Asp Gln Arg Thr Arg
   85 90 95
   Lys Thr Gly Glu Ala Phe Leu Ala Gly Leu Ala Pro Gln Cys Asp Leu
   100 105 110
   Ala Ile His His Gln Gln Asn Ile Gln Gln Ala Asp Pro Leu Phe His
   115 120 125
   Pro Val Lys Ala Gly Ile Cys Ser Met Asp Lys Ser Gln Ala His Ala
   130 135 140
   Ala Val Glu Lys Gln Ala Gly Thr Pro Ile Glu Thr Leu Asn Gln Arg
   145 150 155 160
   Tyr Gln Ala Ser Leu Ala Leu Met Ser Ser Val Leu Asp Phe Pro Lys
   165 170 175
   Ser Pro Tyr Cys Gln Gln His Asn Ile Gly Lys Leu Cys Asp Phe Ser
   180 185 190
   Gln Ala Met Pro Ser Arg Leu Ala Ile Asn Asp Asp Gly Asn Lys Val
   195 200 205
   Ala Leu Glu Gly Ala Val Gly Leu Ser Ser Thr Leu Ala Glu Ile Phe
   210 215 220
   Leu Leu Glu His Ala Gln Gly Met Pro Lys Val Ala Trp Gly Asn Ile
   225 230 235 240
   His Thr Glu Gln Gln Trp Asp Ser Leu Leu Lys Leu His Asn Ala Gln
   245 250 255
   Phe Asp Leu Met Ser Arg Thr Pro Tyr Ile Ala Lys His Asn Gly Thr
   260 265 270
   Pro Leu Leu Gln Thr Ile Ala His Ala Leu Gly Ser Asn Ile Ala Ser
   275 280 285
   Arg Pro Leu Pro Asp Ile Ser Pro Asp Asn Lys Ile Leu Phe Ile Ala
   290 295 300
   Gly His Asp Thr Asn Ile Ala Asn Ile Ser Gly Met Leu Gly Met Thr
   305 310 315 320
   Trp Thr Leu Pro Gly Gln Pro Asp Asn Thr Pro Pro Gly Gly Gly Leu
                   325                 330                 335
   Val Phe Glu Leu Trp Gln Asn Pro Asp Asn His Gln Gln Tyr Val Ala
               340                 345                 350
   Val Lys Met Phe Tyr Gln Thr Met Asp Gln Leu Arg Asn Ser Glu Lys
           355                 360                 365
   Leu Asp Leu Lys Ser His Pro Ala Gly Ile Val Pro Ile Glu Ile Glu
       370                 375                 380
   Gly Cys Glu Asn Ile Gly Thr Asp Lys Leu Cys Gln Leu Asp Thr Phe
   385                 390                 395                 400
   Gln Lys Arg Val Ala Gln Val Ile Glu Pro Ala Cys His Ile
                   405                 410
<210> 19
   <211> 1245
   <212> DNA
   <213> Artificial Sequence
<220>
   <223> synthetic construct
<400> 19
   agtgataccg cccctgctgg gttccagttg gaaaaggttg ttatcctaag cagacatggc      60
   gtacgcgcgc caaccaaaat gacacaaacg atgcgcaacg tcacacctca ccagtggcct     120
   gaatggccgg taaaactcgg ctatatcacg ccccgcggtg aacatctgat tagcctgatg     180
   ggcggttttt atcgagagcg ctttcagcaa caaggcttat tacctaagga taactgtcct     240
   acaccagatg ccgtgtatgt ttgggcagac gtcgatcaac gcacacgtaa aaccggcgag     300
   gccttcttag cgggtcttgc tccccagtgt gatttagcga tccaccatca gcaaaacatt     360
   cagcaggccg atccgctgtt ccatcctgtg aaagccggta tctgttcgat ggataaatca     420
   caggcacacg ccgccgttga aaagcaggca ggcacaccga ttgagacgct caatcaacgc     480
   tatcaagcat ctttagcgct gatgagttcg gtactcgatt ttccaaaatc cccctattgt     540
   cagcagcaca acattggcaa actctgcgat ttttcacagg cgatgcctag caggctggcg     600
   ataaatgacg acggtaataa agtggctctc gaaggtgccg tgggactttc atcgacgttg     660
   gctgaaattt tcctgctgga acacgctcag ggaatgccta aagtggcttg ggggaatatt     720
   cacactgagc agcaatggga ctctctgtta aaattgcata atgcgcagtt tgacttgatg     780
   tcgcgcacgc cctatatcgc caagcataac ggtactccac tgctgcaaac catcgcacac     840
   gcactgggtt ccaatatcgc gagtcgccca ctgccggata tttcgccaga caataagatc     900
   ctgtttattg ccggtcacga caccaatatt gccaatattt ctggcatgct agggatgaca     960
   tggacacttc cgggacagcc cgataacacc ccgccgggtg gggggctggt gtttgaacta    1020
   tggcagaatc cagataacca tcagcaatat gtcgcagtta agatgttcta tcaaacaatg    1080
   gatcagttac gaaatagtga aaagttagac ctgaaaagtc atccagccgg tattgttccc    1140
   attgagatcg aaggttgtga gaacatcggt acagacaaac tttgccagct tgataccttc    1200
   caaaagagag tggctcaggt gattgaacct gcatgccata tttaa                    1245
<210> 20
   <211> 6729
   <212> DNA
   <213> Artificial Sequence
<220>
   <223> synthetic construct
<400> 20
   agcttgcggc cgcactcgag caccaccacc accaccactg agatccggct gctaacaaag 60
   cccgaaagga agctgagttg gctgctgcca ccgctgagca ataactagca taaccccttg 120
   gggcctctaa acgggtcttg aggggttttt tgctgaaagg aggaactata tccggattgg 180
   cgaatgggac gcgccctgta gcggcgcatt aagcgcggcg ggtgtggtgg ttacgcgcag 240
   cgtgaccgct acacttgcca gcgccctagc gcccgctcct ttcgctttct tcccttcctt 300
   tctcgccacg ttcgccggct ttccccgtca agctctaaat cgggggctcc ctttagggtt 360
   ccgatttagt gctttacggc acctcgaccc caaaaaactt gattagggtg atggttcacg 420
   tagtgggcca tcgccctgat agacggtttt tcgccctttg acgttggagt ccacgttctt 480
   taatagtgga ctcttgttcc aaactggaac aacactcaac cctatctcgg tctattcttt 540
   tgatttataa gggattttgc cgatttcggc ctattggtta aaaaatgagc tgatttaaca 600
   aaaatttaac gcgaatttta acaaaatatt aacgtttaca atttcaggtg gcacttttcg 660
   gggaaatgtg cgcggaaccc ctatttgttt atttttctaa atacattcaa atatgtatcc 720
   gctcatgaga caataaccct gataaatgct tcaataatat tgaaaaagga agagtatgag 780
   tattcaacat ttccgtgtcg cccttattcc cttttttgcg gcattttgcc ttcctgtttt 840
   tgctcaccca gaaacgctgg tgaaagtaaa agatgctgaa gatcagttgg gtgcacgagt 900
   gggttacatc gaactggatc tcaacagcgg taagatcctt gagagttttc gccccgaaga 960
   acgttttcca atgatgagca cttttaaagt tctgctatgt ggcgcggtat tatcccgtat 1020
   tgacgccggg caagagcaac tcggtcgccg catacactat tctcagaatg acttggttga 1080
   gtactcacca gtcacagaaa agcatcttac ggatggcatg acagtaagag aattatgcag 1140
   tgctgccata accatgagtg ataacactgc ggccaactta cttctgacaa cgatcggagg 1200
   accgaaggag ctaaccgctt ttttgcacaa catgggggat catgtaactc gccttgatcg 1260
   ttgggaaccg gagctgaatg aagccatacc aaacgacgag cgtgacacca cgatgcctgc 1320
   agcaatggca acaacgttgc gcaaactatt aactggcgaa ctacttactc tagcttcccg 1380
   gcaacaatta atagactgga tggaggcgga taaagttgca ggaccacttc tgcgctcggc 1440
   ccttccggct ggctggttta ttgctgataa atctggagcc ggtgagcgtg ggtctcgcgg 1500
   tatcattgca gcactggggc cagatggtaa gccctcccgt atcgtagtta tctacacgac 1560
   ggggagtcag gcaactatgg atgaacgaaa tagacagatc gctgagatag gtgcctcact 1620
   gattaagcat tggtaactgt cagaccaagt ttactcatat atactttaga ttgatttaaa 1680
   acttcatttt taatttaaaa ggatctaggt gaagatcctt tttgataatc tcatgaccaa 1740
   aatcccttaa cgtgagtttt cgttccactg agcgtcagac cccgtagaaa agatcaaagg 1800
   atcttcttga gatccttttt ttctgcgcgt aatctgctgc ttgcaaacaa aaaaaccacc 1860
   gctaccagcg gtggtttgtt tgccggatca agagctacca actctttttc cgaaggtaac 1920
   tggcttcagc agagcgcaga taccaaatac tgtccttcta gtgtagccgt agttaggcca 1980
   ccacttcaag aactctgtag caccgcctac atacctcgct ctgctaatcc tgttaccagt 2040
   ggctgctgcc agtggcgata agtcgtgtct taccgggttg gactcaagac gatagttacc 2100
   ggataaggcg cagcggtcgg gctgaacggg gggttcgtgc acacagccca gcttggagcg 2160
   aacgacctac accgaactga gatacctaca gcgtgagcta tgagaaagcg ccacgcttcc 2220
   cgaagggaga aaggcggaca ggtatccggt aagcggcagg gtcggaacag gagagcgcac 2280
   gagggagctt ccagggggaa acgcctggta tctttatagt cctgtcgggt ttcgccacct 2340
   ctgacttgag cgtcgatttt tgtgatgctc gtcagggggg cggagcctat ggaaaaacgc 2400
   cagcaacgcg gcctttttac ggttcctggc cttttgctgg ccttttgctc acatgttctt 2460
   tcctgcgtta tcccctgatt ctgtggataa ccgtattacc gcctttgagt gagctgatac 2520
   cgctcgccgc agccgaacga ccgagcgcag cgagtcagtg agcgaggaag cggaagagcg 2580
   cctgatgcgg tattttctcc ttacgcatct gtgcggtatt tcacaccgca tatatggtgc 2640
   actctcagta caatctgctc tgatgccgca tagttaagcc agtatacact ccgctatcgc 2700
   tacgtgactg ggtcatggct gcgccccgac acccgccaac acccgctgac gcgccctgac 2760
   gggcttgtct gctcccggca tccgcttaca gacaagctgt gaccgtctcc gggagctgca 2820
   tgtgtcagag gttttcaccg tcatcaccga aacgcgcgag gcagctgcgg taaagctcat 2880
   cagcgtggtc gtgaagcgat tcacagatgt ctgcctgttc atccgcgtcc agctcgttga 2940
   gtttctccag aagcgttaat gtctggcttc tgataaagcg ggccatgtta agggcggttt 3000
   tttcctgttt ggtcactgat gcctccgtgt aagggggatt tctgttcatg ggggtaatga 3060
   taccgatgaa acgagagagg atgctcacga tacgggttac tgatgatgaa catgcccggt 3120
   tactggaacg ttgtgagggt aaacaactgg cggtatggat gcggcgggac cagagaaaaa 3180
   tcactcaggg tcaatgccag cgcttcgtta atacagatgt aggtgttcca cagggtagcc 3240
   agcagcatcc tgcgatgcag atccggaaca taatggtgca gggcgctgac ttccgcgttt 3300
   ccagacttta cgaaacacgg aaaccgaaga ccattcatgt tgttgctcag gtcgcagacg 3360
   ttttgcagca gcagtcgctt cacgttcgct cgcgtatcgg tgattcattc tgctaaccag 3420
   taaggcaacc ccgccagcct agccgggtcc tcaacgacag gagcacgatc atgcgcaccc 3480
   gtggggccgc catgccggcg ataatggcct gcttctcgcc gaaacgtttg gtggcgggac 3540
   cagtgacgaa ggcttgagcg agggcgtgca agattccgaa taccgcaagc gacaggccga 3600
   tcatcgtcgc gctccagcga aagcggtcct cgccgaaaat gacccagagc gctgccggca 3660
   cctgtcctac gagttgcatg ataaagaaga cagtcataag tgcggcgacg atagtcatgc 3720
   cccgcgccca ccggaaggag ctgactgggt tgaaggctct caagggcatc ggtcgagatc 3780
   ccggtgccta atgagtgagc taacttacat taattgcgtt gcgctcactg cccgctttcc 3840
   agtcgggaaa cctgtcgtgc cagctgcatt aatgaatcgg ccaacgcgcg gggagaggcg 3900
   gtttgcgtat tgggcgccag ggtggttttt cttttcacca gtgagacggg caacagctga 3960
   ttgcccttca ccgcctggcc ctgagagagt tgcagcaagc ggtccacgct ggtttgcccc 4020
   agcaggcgaa aatcctgttt gatggtggtt aacggcggga tataacatga gctgtcttcg 4080
   gtatcgtcgt atcccactac cgagatatcc gcaccaacgc gcagcccgga ctcggtaatg 4140
   gcgcgcattg cgcccagcgc catctgatcg ttggcaacca gcatcgcagt gggaacgatg 4200
   ccctcattca gcatttgcat ggtttgttga aaaccggaca tggcactcca gtcgccttcc 4260
   cgttccgcta tcggctgaat ttgattgcga gtgagatatt tatgccagcc agccagacgc 4320
   agacgcgccg agacagaact taatgggccc gctaacagcg cgatttgctg gtgacccaat 4380
   gcgaccagat gctccacgcc cagtcgcgta ccgtcttcat gggagaaaat aatactgttg 4440
   atgggtgtct ggtcagagac atcaagaaat aacgccggaa cattagtgca ggcagcttcc 4500
   acagcaatgg catcctggtc atccagcgga tagttaatga tcagcccact gacgcgttgc 4560
   gcgagaagat tgtgcaccgc cgctttacag gcttcgacgc cgcttcgttc taccatcgac 4620
   accaccacgc tggcacccag ttgatcggcg cgagatttaa tcgccgcgac aatttgcgac 4680
   ggcgcgtgca gggccagact ggaggtggca acgccaatca gcaacgactg tttgcccgcc 4740
   agttgttgtg ccacgcggtt gggaatgtaa ttcagctccg ccatcgccgc ttccactttt 4800
   tcccgcgttt tcgcagaaac gtggctggcc tggttcacca cgcgggaaac ggtctgataa 4860
   gagacaccgg catactctgc gacatcgtat aacgttactg gtttcacatt caccaccctg 4920
   aattgactct cttccgggcg ctatcatgcc ataccgcgaa aggttttgcg ccattcgatg 4980
   gtgtccggga tctcgacgct ctcccttatg cgactcctgc attaggaagc agcccagtag 5040
   taggttgagg ccgttgagca ccgccgccgc aaggaatggt gcatgcaagg agatggcgcc 5100
   caacagtccc ccggccacgg ggcctgccac catacccacg ccgaaacaag cgctcatgag 5160
   cccgaagtgg cgagcccgat cttccccatc ggtgatgtcg gcgatatagg cgccagcaac 5220
   cgcacctgtg gcgccggtga tgccggccac gatgcgtccg gcgtagagga tcgagatctc 5280
   gatcccgcga aattaatacg actcactata ggggaattgt gagcggataa caattcccct 5340
   ctagaaataa ttttgtttaa ctttaagaag gagatataca tatgatgaca atctctctgt 5400
   ttaaccgtaa taaacccgct attgcacagc gtattttatg tcctctgatc gtggctttat 5460
   tctcaggttt accggcatac gccagtgata ccgcccctgc tgggttccag ttggaaaagg 5520
   ttgttatcct aagcagacat ggcgtacgcg cgccaaccaa aatgacacaa acgatgcgca 5580
   acgtcacacc tcaccagtgg cctgaatggc cggtaaaact cggctatatc acgccccgcg 5640
   gtgaacatct gattagcctg atgggcggtt tttatcgaga gcgctttcag caacaaggct 5700
   tattacctaa ggataactgt cctacaccag atgccgtgta tgtttgggca gacgtcgatc 5760
   aacgcacacg taaaaccggc gaggccttct tagcgggtct tgctccccag tgtgatttag 5820
   cgatccacca tcagcaaaac attcagcagg ccgatccgct gttccatcct gtgaaagccg 5880
   gtatctgttc gatggataaa tcacaggcac acgccgccgt tgaaaagcag gcaggcacac 5940
   cgattgagac gctcaatcaa cgctatcaag catctttagc gctgatgagt tcggtactcg 6000
   attttccaaa atccccctat tgtcagcagc acaacattgg caaactctgc gatttttcac 6060
   aggcgatgcc tagcaggctg gcgataaatg acgacggtaa taaagtggct ctcgaaggtg 6120
   ccgtgggact ttcatcgacg ttggctgaaa ttttcctgct ggaacacgct cagggaatgc 6180
   ctaaagtggc ttgggggaat attcacactg agcagcaatg ggactctctg ttaaaattgc 6240
   ataatgcgca gtttgacttg atgtcgcgca cgccctatat cgccaagcat aacggtactc 6300
   cactgctgca aaccatcgca cacgcactgg gttccaatat cgcgagtcgc ccactgccgg 6360
   atatttcgcc agacaataag atcctgttta ttgccggtca cgacaccaat attgccaata 6420
   tttctggcat gctagggatg acatggacac ttccgggaca gcccgataac accccgccgg 6480
   gtggggggct ggtgtttgaa ctatggcaga atccagataa ccatcagcaa tatgtcgcag 6540
   ttaagatgtt ctatcaaaca atggatcagt tacgaaatag tgaaaagtta gacctgaaaa 6600
   gtcatccagc cggtattgtt cccattgaga tcgaaggttg tgagaacatc ggtacagaca 6660
   aactttgcca gcttgatacc ttccaaaaga gagtggctca ggtgattgaa cctgcatgcc 6720
   atatttaaa 6729
<210> 21
   <211> 46
   <212> DNA
   <213> Artificial Sequence
<220>
   <223> primer
<400> 21
   ctatggatcc gcatcatcat catcatcaca gtgataccgc ccctgc 46
<210> 22
   <211> 6738
   <212> DNA
   <213> Artificial Sequence
<220>
   <223> synthetic construct
<400> 22
   gagatataca tatgaaatac ctgctgccga ccgctgctgc tggtctgctg ctcctcgctg 60
   cccagccggc gatggccatg gatatcggaa ttaattcgga tccgcatcat catcatcatc 120
   acagtgatac cgcccctgct gggttccagt tggaaaaggt tgttatccta agcagacatg 180
   gcgtacgcgc gccaaccaaa atgacacaaa cgatgcgcaa cgtcacacct caccagtggc 240
   ctgaatggcc ggtaaaactc ggctatatca cgccccgcgg tgaacatctg attagcctga 300
   tgggcggttt ttatcgagag cgctttcagc aacaaggctt attacctaag gataactgtc 360
   ctacaccaga tgccgtgtat gtttgggcag acgtcgatca acgcacacgt aaaaccggcg 420
   aggccttctt agcgggtctt gctccccagt gtgatttagc gatccaccat cagcaaaaca 480
   ttcagcaggc cgatccgctg ttccatcctg tgaaagccgg tatctgttcg atggataaat 540
   cacaggcaca cgccgccgtt gaaaagcagg caggcacacc gattgagacg ctcaatcaac 600
   gctatcaagc atctttagcg ctgatgagtt cggtactcga ttttccaaaa tccccctatt 660
   gtcagcagca caacattggc aaactctgcg atttttcaca ggcgatgcct agcaggctgg 720
   cgataaatga cgacggtaat aaagtggctc tcgaaggtgc cgtgggactt tcatcgacgt 780
   tggctgaaat ttccctgctg gaacacgctc agggaatgcc taaagtggct tgggggaata 840
   ttcacactga gcagcaatgg gactctctgt taaaattgca taatgcgcag tttgacttga 900
   tgtcgcgcac gccctatatc gccaagcata acggtactcc actgctgcaa accatcgcac 960
   acgcactggg ttccaatatc gcgagtcgcc cactgccgga tatttcgcca gacaataaga 1020
   tcctgtttat tgccggtcac gacaccaata ttgccaatat ttctggcatg ctagggatga 1080
   catggacact tccgggacag cccgataaca ccccgccggg tgggggcctg gtgtttgaac 1140
   tatggcagaa tccagataac catcagcaat atgtcgcagt taagatgttc tatcaaacaa 1200
   tggatcagtt acgaaatagt gaaaagttag acctgaaaag tcatccagcc ggtattgttc 1260
   ccattgagat cgaaggttgt gagaacatcg gtacagacaa actttgccag cttgatacct 1320
   tccaaaagag agtggctcag gtgattgaac ctgcatgcca tatttaaaag cttgcggccg 1380
   cactcgagca ccaccaccac caccactgag atccggctgc taacaaagcc cgaaaggaag 1440
   ctgagttggc tgctgccacc gctgagcaat aactagcata accccttggg gcctctaaac 1500
   gggtcttgag gggttttttg ctgaaaggag gaactatatc cggattggcg aatgggacgc 1560
   gccctgtagc ggcgcattaa gcgcggcggg tgtggtggtt acgcgcagcg tgaccgctac 1620
   acttgccagc gccctagcgc ccgctccttt cgctttcttc ccttcctttc tcgccacgtt 1680
   cgccggcttt ccccgtcaag ctctaaatcg ggggctccct ttagggttcc gatttagtgc 1740
   tttacggcac ctcgacccca aaaaacttga ttagggtgat ggttcacgta gtgggccatc 1800
   gccctgatag acggtttttc gccctttgac gttggagtcc acgttcttta atagtggact 1860
   cttgttccaa actggaacaa cactcaaccc tatctcggtc tattcttttg atttataagg 1920
   gattttgccg atttcggcct attggttaaa aaatgagctg atttaacaaa aatttaacgc 1980
   gaattttaac aaaatattaa cgtttacaat ttcaggtggc acttttcggg gaaatgtgcg 2040
   cggaacccct atttgtttat ttttctaaat acattcaaat atgtatccgc tcatgagaca 2100
   ataaccctga taaatgcttc aataatattg aaaaaggaag agtatgagta ttcaacattt 2160
   ccgtgtcgcc cttattccct tttttgcggc attttgcctt cctgtttttg ctcacccaga 2220
   aacgctggtg aaagtaaaag atgctgaaga tcagttgggt gcacgagtgg gttacatcga 2280
   actggatctc aacagcggta agatccttga gagttttcgc cccgaagaac gttttccaat 2340
   gatgagcact tttaaagttc tgctatgtgg cgcggtatta tcccgtattg acgccgggca 2400
   agagcaactc ggtcgccgca tacactattc tcagaatgac ttggttgagt actcaccagt 2460
   cacagaaaag catcttacgg atggcatgac agtaagagaa ttatgcagtg ctgccataac 2520
   catgagtgat aacactgcgg ccaacttact tctgacaacg atcggaggac cgaaggagct 2580
   aaccgctttt ttgcacaaca tgggggatca tgtaactcgc cttgatcgtt gggaaccgga 2640
   gctgaatgaa gccataccaa acgacgagcg tgacaccacg atgcctgcag caatggcaac 2700
   aacgttgcgc aaactattaa ctggcgaact acttactcta gcttcccggc aacaattaat 2760
   agactggatg gaggcggata aagttgcagg accacttctg cgctcggccc ttccggctgg 2820
   ctggtttatt gctgataaat ctggagccgg tgagcgtggg tctcgcggta tcattgcagc 2880
   actggggcca gatggtaagc cctcccgtat cgtagttatc tacacgacgg ggagtcaggc 2940
   aactatggat gaacgaaata gacagatcgc tgagataggt gcctcactga ttaagcattg 3000
   gtaactgtca gaccaagttt actcatatat actttagatt gatttaaaac ttcattttta 3060
   atttaaaagg atctaggtga agatcctttt tgataatctc atgaccaaaa tcccttaacg 3120
   tgagttttcg ttccactgag cgtcagaccc cgtagaaaag atcaaaggat cttcttgaga 3180
   tccttttttt ctgcgcgtaa tctgctgctt gcaaacaaaa aaaccaccgc taccagcggt 3240
   ggtttgtttg ccggatcaag agctaccaac tctttttccg aaggtaactg gcttcagcag 3300
   agcgcagata ccaaatactg tccttctagt gtagccgtag ttaggccacc acttcaagaa 3360
   ctctgtagca ccgcctacat acctcgctct gctaatcctg ttaccagtgg ctgctgccag 3420
   tggcgataag tcgtgtctta ccgggttgga ctcaagacga tagttaccgg ataaggcgca 3480
   gcggtcgggc tgaacggggg gttcgtgcac acagcccagc ttggagcgaa cgacctacac 3540
   cgaactgaga tacctacagc gtgagctatg agaaagcgcc acgcttcccg aagggagaaa 3600
   ggcggacagg tatccggtaa gcggcagggt cggaacagga gagcgcacga gggagcttcc 3660
   agggggaaac gcctggtatc tttatagtcc tgtcgggttt cgccacctct gacttgagcg 3720
   tcgatttttg tgatgctcgt caggggggcg gagcctatgg aaaaacgcca gcaacgcggc 3780
   ctttttacgg ttcctggcct tttgctggcc ttttgctcac atgttctttc ctgcgttatc 3840
   ccctgattct gtggataacc gtattaccgc ctttgagtga gctgataccg ctcgccgcag 3900
   ccgaacgacc gagcgcagcg agtcagtgag cgaggaagcg gaagagcgcc tgatgcggta 3960
   ttttctcctt acgcatctgt gcggtatttc acaccgcata tatggtgcac tctcagtaca 4020
   atctgctctg atgccgcata gttaagccag tatacactcc gctatcgcta cgtgactggg 4080
   tcatggctgc gccccgacac ccgccaacac ccgctgacgc gccctgacgg gcttgtctgc 4140
   tcccggcatc cgcttacaga caagctgtga ccgtctccgg gagctgcatg tgtcagaggt 4200
   tttcaccgtc atcaccgaaa cgcgcgaggc agctgcggta aagctcatca gcgtggtcgt 4260
   gaagcgattc acagatgtct gcctgttcat ccgcgtccag ctcgttgagt ttctccagaa 4320
   gcgttaatgt ctggcttctg ataaagcggg ccatgttaag ggcggttttt tcctgtttgg 4380
   tcactgatgc ctccgtgtaa gggggatttc tgttcatggg ggtaatgata ccgatgaaac 4440
   gagagaggat gctcacgata cgggttactg atgatgaaca tgcccggtta ctggaacgtt 4500
   gtgagggtaa acaactggcg gtatggatgc ggcgggacca gagaaaaatc actcagggtc 4560
   aatgccagcg cttcgttaat acagatgtag gtgttccaca gggtagccag cagcatcctg 4620
   cgatgcagat ccggaacata atggtgcagg gcgctgactt ccgcgtttcc agactttacg 4680
   aaacacggaa accgaagacc attcatgttg ttgctcaggt cgcagacgtt ttgcagcagc 4740
   agtcgcttca cgttcgctcg cgtatcggtg attcattctg ctaaccagta aggcaacccc 4800
   gccagcctag ccgggtcctc aacgacagga gcacgatcat gcgcacccgt ggggccgcca 4860
   tgccggcgat aatggcctgc ttctcgccga aacgtttggt ggcgggacca gtgacgaagg 4920
   cttgagcgag ggcgtgcaag attccgaata ccgcaagcga caggccgatc atcgtcgcgc 4980
   tccagcgaaa gcggtcctcg ccgaaaatga cccagagcgc tgccggcacc tgtcctacga 5040
   gttgcatgat aaagaagaca gtcataagtg cggcgacgat agtcatgccc cgcgcccacc 5100
   ggaaggagct gactgggttg aaggctctca agggcatcgg tcgagatccc ggtgcctaat 5160
   gagtgagcta acttacatta attgcgttgc gctcactgcc cgctttccag tcgggaaacc 5220
   tgtcgtgcca gctgcattaa tgaatcggcc aacgcgcggg gagaggcggt ttgcgtattg 5280
   ggcgccaggg tggtttttct tttcaccagt gagacgggca acagctgatt gcccttcacc 5340
   gcctggccct gagagagttg cagcaagcgg tccacgctgg tttgccccag caggcgaaaa 5400
   tcctgtttga tggtggttaa cggcgggata taacatgagc tgtcttcggt atcgtcgtat 5460
   cccactaccg agatatccgc accaacgcgc agcccggact cggtaatggc gcgcattgcg 5520
   cccagcgcca tctgatcgtt ggcaaccagc atcgcagtgg gaacgatgcc ctcattcagc 5580
   atttgcatgg tttgttgaaa accggacatg gcactccagt cgccttcccg ttccgctatc   5640
   ggctgaattt gattgcgagt gagatattta tgccagccag ccagacgcag acgcgccgag   5700
   acagaactta atgggcccgc taacagcgcg atttgctggt gacccaatgc gaccagatgc   5760
   tccacgccca gtcgcgtacc gtcttcatgg gagaaaataa tactgttgat gggtgtctgg   5820
   tcagagacat caagaaataa cgccggaaca ttagtgcagg cagcttccac agcaatggca   5880
   tcctggtcat ccagcggata gttaatgatc agcccactga cgcgttgcgc gagaagattg   5940
   tgcaccgccg ctttacaggc ttcgacgccg cttcgttcta ccatcgacac caccacgctg   6000
   gcacccagtt gatcggcgcg agatttaatc gccgcgacaa tttgcgacgg cgcgtgcagg   6060
   gccagactgg aggtggcaac gccaatcagc aacgactgtt tgcccgccag ttgttgtgcc   6120
   acgcggttgg gaatgtaatt cagctccgcc atcgccgctt ccactttttc ccgcgttttc   6180
   gcagaaacgt ggctggcctg gttcaccacg cgggaaacgg tctgataaga gacaccggca   6240
   tactctgcga catcgtataa cgttactggt ttcacattca ccaccctgaa ttgactctct   6300
   tccgggcgct atcatgccat accgcgaaag gttttgcgcc attcgatggt gtccgggatc   6360
   tcgacgctct cccttatgcg actcctgcat taggaagcag cccagtagta ggttgaggcc   6420
   gttgagcacc gccgccgcaa ggaatggtgc atgcaaggag atggcgccca acagtccccc   6480
   ggccacgggg cctgccacca tacccacgcc gaaacaagcg ctcatgagcc cgaagtggcg   6540
   agcccgatct tccccatcgg tgatgtcggc gatataggcg ccagcaaccg cacctgtggc   6600
   gccggtgatg ccggccacga tgcgtccggc gtagaggatc gagatctcga tcccgcgaaa   6660
   ttaatacgac tcactatagg ggaattgtga gcggataaca attcccctct agaaataatt   6720
   ttgtttaact ttaagaag                                                 6738
<210> 23
   <211> 10331
   <212> DNA
   <213> Artificial Sequence
<220>
   <223> synthetic construct
<400> 23
   aagcttgcat gagcatgtca cagtcgaatt ctggggtcac gcggtgcttg agggcgaata      60
   cggctccatc ggtgagtaac ctctctctta ctaccacgga aacatcactg acgtaaccag     120
   gacccggcgg cttatccatc atgggaaaca acacctacaa atccgccaga attctctcgg     180
   aagaatataa cctctactac tccgtctggt gcgacggtga ccacgagctg tacgatctct     240
   cagtaagtgc caaccggttc ccgccactat cgtaaaaaca aaaaatctaa caacaccaga     300
   cggaccccta ccaaatgaac aacatctaca cccaacaaga caacatccac ctcctaagca     360
   gacctctatc cagcgtgatt gatcgtatcg acgctctcct tctggttctg aaatcctgca     420
   agggtaacac atgcatccag ccgtggcggg tcctccaccc cgacgggtcc gtagagagcc 480
   tcaaagatgc actgcaggtg aaatacgatt ccttttacac caaccagccc aaggtgtcgt 540
   attcagtatg tgaacccggg tacatcattg aggctgaggg gccccaggtc ggattgcagt 600
   atagagatgg gctgagttgg gaggcgtgga cttgacgatt ccgtcaagta tgagtatggg 660
   tacgaataat gagcgttatt gctatgtatt tttatagata gtttatttat atatcatgac 720
   taaacttgag agccatggaa tcaatgaaat gacatggcga gtgtagatca cgatagtcat 780
   agtagccgaa gtgggcggat agccaagaat aacaccagaa tcagataaca ggaacatcac 840
   aaccgatcac accatagata atatccaaag aagtttaaat agccgagaca aagagaatag 900
   agacaagata catggaacaa gaaaggtaca cccggtagat aaaccctggg acgggcccga 960
   gtccttaccc atagatcaat cccacgggaa caaaaccaaa gtcaacaacc accaccacca 1020
   ttaccacaac cgcatcaata gaaccggtga aaaatgacac catcgaatcc ttcaccctaa 1080
   gtaaagccct gtacgttgca tatcgcttaa gcacaaaagt agtagaatag atatgagccc 1140
   gcacgcgcgg ccaacgatcc aaactagccc tgacatcaaa gccagcggcg attgcgccat 1200
   caagcccccg tctcacttca tagtggaatt gcgggtcacc tcactgattg actgtctgtc 1260
   tagacacact cacccacgca tgctgtctgt gcccagaacg tggactttgg ctctgccgag 1320
   ctagaggatc aaatataagt agattggatg taggcccgta ttttttttat ttcgtgtgac 1380
   tcggagattt tatgcgttgt gttgttgggc ggaaaaagaa atatactttc tttttgttct 1440
   tttctttttc tctctattgc ttgccttgga tatcccttgc atacggtcgg ttgctgattg 1500
   actaagggtg ctgtcttgtg tcactgaact gctgctcaac ctctgtctgg tattcctgtt 1560
   gtcgtgatgg tggggaaaca gttcgagttc gaggaccaga gggatggcat cgtgcctccc 1620
   ttggaggaaa agaaggtcgt cgatgaggtc tataccgata atgatgttgc gtcggaggag 1680
   attgtcaagg actgggatga taaggaggag ggcaagctgc ggaggaagtg agtcgtcact 1740
   gttttcattc actgccatat aggttcaagc atatactgac tggtatatag gatcgatatc 1800
   atcctcatcc ccattctcgc tctcgctttc ttcggcctcc agattgatcg cggcaatatc 1860
   agcgcagctc ttacctccac tatcaccgaa gacctaggtg tcaccacgaa ccaaatcaat 1920
   attggaaccc agttgctttc ggctggtatt gtcatcaccg agatcccgtc aaatattata 1980
   cttcagcgca tcggtcccca ggtctggttg tcggcacagc tgatcgcttg gggtctggtt 2040
   ggcacattcc aggcttttgt acagtcgtac ccggcgtatc tggccacgag gttgttgctg 2100
   gggctgttgg agggagggtt tattcctggt ttgtctggtc gtgcgccttg gtctatggtg 2160
   gtagcgctaa caatgggttt ggtacaggtg ccctgtacta tctctcgaca tggtataaac 2220
   gtcctgagac gagtttccgg accactctgt tcttctatgg gcagatgttt gccggtgcga 2280
   cctcgagcgg ccgcttcgag gattgcctga acattgacat tcggcgtccg gccgggacca 2340
   ccgcggactc gaagctgcct gtgctggtct ggatctttgg cggaggcttt gaacttggtt 2400
   caaaggcgat gtatgatggt acaacgatgg tatcatcgtc gatagacaag aacatgccta 2460
   tcgtgtttgt agcaatgaat tatcgcgtgg gaggtttcgg gttcttgccc ggaaaggaga 2520
   tcctggagga cgggtccgcg aacctagggc tcctggacca acgccttgcc ctgcagtggg 2580
   ttgccgacaa catcgaggcc tttggtggag acccggacaa ggtgacgatt tggggagaat 2640
   cagcaggagc catttccgtt tttgatcaga tgatcttgta cgacggaaac atcacttaca 2700
   aggataagcc cttgttccgg ggggccatca tggactccgg tagtgttgtt cccgcagacc 2760
   ccgtcgatgg ggtcaaggga cagcaagtat atgatgcggt agtggaatct gcaggctgtt 2820
   cctcttctaa cgacacccta gcttgtctgc gtgaactaga ctacaccgac ttcctcaatg 2880
   cggcaaactc cgtgccaggc attttaagct accattctgt ggcgttatca tatgtgcctc 2940
   gaccggacgg gacggcgttg tcggcatcac cggacgtttt gggcaaagca gggaaatatg 3000
   ctcgggtccc gttcatcgtg ggcgaccaag aggatgaggg gaccttattc gccttgtttc 3060
   agtccaacat tacgacgatc gacgaggtgg tcgactacct ggcctcatac ttcttctatg 3120
   acgctagccg agagcagctt gaagaactag tggccctgta cccagacacc accacgtacg 3180
   ggtctccgtt caggacaggc gcggccaaca actggtatcc gcaatttaag cgattggccg 3240
   ccattctcgg cgacttggtc ttcaccatta cccggcgggc attcctctcg tatgcagagg 3300
   aaatctcccc tgatcttccg aactggtcgt acctggcgac ctatgactat ggcaccccag 3360
   ttctggggac cttccacgga agtgacctgc tgcaggtgtt ctatgggatc aagccaaact 3420
   atgcagctag ttctagccac acgtactatc tgagctttgt gtatacgctg gatccgaact 3480
   ccaaccgggg ggagtacatt gagtggccgc agtggaagga atcgcggcag ttgatgaatt 3540
   tcggagcgaa cgacgccagt ctccttacgg atgatttccg caacgggaca tatgagttca 3600
   tcctgcagaa taccgcggcg ttccacatct gatgccattg gcggaggggt ccggacggtc 3660
   aggaacttag ccttatgaga tgaatgatgg acgtgtctgg cctcggaaaa ggatatatgg 3720
   ggatcatgat agtactagcc atattaatga agggcatata ccacgcgttg gacctgcgtt 3780
   atagcttccc gttagttata gtaccatcgt tataccagcc aatcaagtca ccacgcacga 3840
   ccggggacgg cgaatccccg ggaattgaaa gaaattgcat cccaggccag tgaggccagc 3900
   gattggccac ctctccaagg cacagggcca ttctgcagcg ctggtggatt catcgcaatt 3960
   tcccccggcc cggcccgaca ccgctatagg ctggttctcc cacaccatcg gagattcgtc 4020
   gcctaatgtc tcgtccgttc acaagctgaa gagcttgaag tggcgagatg tctctgcagg 4080
   aattcaagct agatgctaag cgatattgca tggcaatatg tgttgatgca tgtgcttctt 4140
   ccttcagctt cccctcgtgc agatgaggtt tggctataaa ttgaagtggt tggtcggggt 4200
   tccgtgaggg gctgaagtgc ttcctccctt ttagacgcaa ctgagagcct gagcttcatc 4260
   cccagcatca ttacacctca gcaatgggcg tctctgctgt tctacttcct ttgtatctcc 4320
   tgtctgggta tgctaagcac cacaatcaaa gtctaataag gaccctccct tccgagggcc 4380
   cctgaagctc ggactgtgtg ggactactga tcgctgacta tctgtgcaga gtcacctccg 4440
   gactggcagt ccccagtgat accgcccctg ctgggttcca gttggaaaag gttgttatcc 4500
   taagcagaca tggcgtacgc gcgccaacca aaatgacaca aacgatgcgc aacgtcacac 4560
   ctcaccagtg gcctgaatgg ccggtaaaac tcggctatat cacgccccgc ggtgaacatc 4620
   tgattagcct gatgggcggt ttttatcgag agcgctttca gcaacaaggc ttattaccta 4680
   aggataactg tcctacacca gatgccgtgt atgtttgggc agacgtcgat caacgcacac 4740
   gtaaaaccgg cgaggccttc ttagcgggtc ttgctcccca gtgtgattta gcgatccacc 4800
   atcagcaaaa cattcagcag gccgatccgc tgttccatcc tgtgaaagcc ggtatctgtt 4860
   cgatggataa atcacaggca cacgccgccg ttgaaaagca ggcaggcaca ccgattgaga 4920
   cgctcaatca acgctatcaa gcatctttag cgctgatgag ttcggtactc gattttccaa 4980
   aatcccccta ttgtcagcag cacaacattg gcaaactctg cgatttttca caggcgatgc 5040
   ctagcaggct ggcgataaat gacgacggta ataaagtggc tctcgaaggt gccgtgggac 5100
   tttcatcgac gttggctgaa attttcctgc tggaacacgc tcagggaatg cctaaagtgg 5160
   cttgggggaa tattcacact gagcagcaat gggactctct gttaaaattg cataatgcgc 5220
   agtttgactt gatgtcgcgc acgccctata tcgccaagca taacggtact ccactgctgc 5280
   aaaccatcgc acacgcactg ggttccaata tcgcgagtcg cccactgccg gatatttcgc 5340
   cagacaataa gatcctgttt attgccggtc acgacaccaa tattgccaat atttctggca 5400
   tgctagggat gacatggaca cttccgggac agcccgataa caccccgccg ggtggggggc 5460
   tggtgtttga actatggcag aatccagata accatcagca atatgtcgca gttaagatgt 5520
   tctatcaaac aatggatcag ttacgaaata gtgaaaagtt agacctgaaa agtcatccag 5580
   ccggtattgt tcccattgag atcgaaggtt gtgagaacat cggtacagac aaactttgcc 5640
   agcttgatac cttccaaaag agagtggctc aggtgattga acctgcatgc catatttaga 5700
   caatcaatcc atttcgctat agttaaagga tggggatgag ggcaattggt tatatgatca 5760
   tgtatgtagt gggtgtgcat aatagtagtg aaatggaagc caagtcatgt gattgtaatc 5820
   gaccgacgga attgaggata tccggaaata cagacaccgt gaaagccatg gtctttcctt 5880
   cgtgtagaag accagacaga cagtccctga tttaccctgc acaaagcact agaaaattag 5940
   cattccatcc ttctctgctt gctctgctga tatcactgtc attcaatgca tagccatgag 6000
   ctcatcttag atccaagcac gtaattccat agccgaggtc cacagtggag cagcaacatt 6060
   ccccatcatt gctttcccca ggggcctccc aacgactaaa tcaagagtat atctctaccg 6120
   tccaatagat cgtcttcgct tcaaaatctt tgacaattcc aagagggtcc ccatccatca 6180
   aacccagttc aataatagcc gagatgcatg gtggagtcaa ttaggcagta ttgctggaat 6240
   gtcggggcca gttccgggtg gtcattggcc gcctgtgatg ccatctgcca ctaaatccga 6300
   tcattgatcc accgcccacg agggcgtctt tgctttttgc gcggcgtcca ggttcaactc 6360
   tctctgcagc tccagtccaa cgctgactga ctagtttacc tactggtctg atcggctcca 6420
   tcagagctat ggcgttatcc cgtgccgttg ctgcgcaatc gctatcttga tcgcaacctt 6480
   gaactcactc ttgttttaat agtgatcttg gtgacggagt gtcggtgagt gacaaccaac 6540
   atcgtgcaag ggagattgat acggaattgt cgctcccatc atgatgttct tgccggcttt 6600
   gttggcccta ttcgtgggat cgatgccctc ctgtgcagca gcaggtactg ctggatgagg 6660
   agccatcggt ctctgcacgc aaacccaact tcctcttcat tctcacggat gatcaggatc 6720
   tccggatgaa ttctccggcg tatatgccgt atacgcaggc gagaatcaag gaaaagggta 6780
   ccgagttctt gaaccatttc gtcactaccg cgctttgctg tccgtcgcgc gtgagtcttt 6840
   ggacgggaag acaggctcat aatactaatg tgacggatgt gaacccgcct tatggtatgg 6900
   acactgcttc gatcggtctt gattcttcag cgtggttaca attgctaatg cggcataggc 6960
   ggatacccca aattcgtcgc tcaaggcttc aacgaaaact tcctccccgt ttggctgcag 7020
   tccgccggtt acaataccta ctacacgggg aagctgttca actcgcacag tgtcgctacc 7080
   tataacgcgc cctttgtgaa cggtttcaat ggctccgact tcctcctcga cccccacaca 7140
   tattcctact ggaatgcgac ataccagcga aaccatgagc ctccgcggag ttacgaggga 7200
   caatatacta cggatgtgat gaaggagaag gcatcgggat tgttggcaga tgcgctggac 7260
   agtgacgcgc cattcttcct gacggtcgcg ccgatcgcac cgcacacgaa catcgatgtg 7320
   gaggggctga gcggtgcggg tggaccgaag atgacagagc cgctgcctgc accgagacat 7380
   gcgcatttgt ttgctgatgc aaaggtgccg cggacgccta atttcaatcc ggacaaggtg 7440
   tgtgatatcc tgacacagtg gtggggacgg gcactgacaa gagtaggatt ctggtgcggg 7500
   gtggatccaa accatggaac tacagaacca gaccgtcatc gactacgaag accatcttta 7560
   tcgccagcgt ctgcgcactt tgcaagccgt cgatgagatg gtggatgcgc tgatcacgca 7620
   gctggaagaa agtgggcaga tcgacaatac ctacatcatt tacagtgctg ataacggcta 7680
   ccacattggc catcaccgtc taccccccgg caagacaact ggctatgaag aggacattcg 7740
   cgtaccattc tacattcgcg gacctggcat tcctgaggga aagagcgttg accgtgtaac 7800
   cacgcacatt gacattgcac ctacactgtt cgagttggct ggggttccct tgcgagagga 7860
   ctttgacggg actccgatgc ccgtgtcgac tagcaagaag acccagtcaa gcttgcatgc 7920
   ctgcaggtcg actctagagg atctgccggt ctccctatag tgagtcgtat taatttcgat 7980
   aagccaggtt aacctgcatt aatgaatcgg ccaacgcgcg gggagaggcg gtttgcgtat 8040
   tgggcgctct tccgcttcct cgctcactga ctcgctgcgc tcggtcgttc ggctgcggcg 8100
   agcggtatca gctcactcaa aggcggtaat acggttatcc acagaatcag gggataacgc 8160
   aggaaagaac atgtgagcaa aaggccagca aaaggccagg aaccgtaaaa aggccgcgtt 8220
   gctggcgttt ttccataggc tccgcccccc tgacgagcat cacaaaaatc gacgctcaag 8280
   tcagaggtgg cgaaacccga caggactata aagataccag gcgtttcccc ctggaagctc 8340
   cctcgtgcgc tctcctgttc cgaccctgcc gcttaccgga tacctgtccg cctttctccc 8400
   ttcgggaagc gtggcgcttt ctcatagctc acgctgtagg tatctcagtt cggtgtaggt 8460
   cgttcgctcc aagctgggct gtgtgcacga accccccgtt cagcccgacc gctgcgcctt 8520
   atccggtaac tatcgtcttg agtccaaccc ggtaagacac gacttatcgc cactggcagc 8580
   agccactggt aacaggatta gcagagcgag gtatgtaggc ggtgctacag agttcttgaa 8640
   gtggtggcct aactacggct acactagaag gacagtattt ggtatctgcg ctctgctgaa 8700
   gccagttacc ttcggaaaaa gagttggtag ctcttgatcc ggcaaacaaa ccaccgctgg 8760
   tagcggtggt ttttttgttt gcaagcagca gattacgcgc agaaaaaaag gatctcaaga 8820
   agatcctttg atcttttcta cggggtctga cgctcagtgg aacgaaaact cacgttaagg 8880
   gattttggtc atgagattat caaaaaggat cttcacctag atccttttaa attaaaaatg 8940
   aagttttaaa tcaatctaaa gtatatatga gtaaacttgg tctgacagtt accaatgctt 9000
   aatcagtgag gcacctatct cagcgatctg tctatttcgt tcatccatag ttgcctgact 9060
   ccccgtcgtg tagataacta cgatacggga gggcttacca tctggcccca gtgctgcaat 9120
   gataccgcga gacccacgct caccggctcc agatttatca gcaataaacc agccagccgg 9180
   aagggccgag cgcagaagtg gtcctgcaac tttatccgcc tccatccagt ctattaattg 9240
   ttgccgggaa gctagagtaa gtagttcgcc agttaatagt ttgcgcaacg ttgttgccat 9300
   tgctacaggc atcgtggtgt cacgctcgtc gtttggtatg gcttcattca gctccggttc 9360
   ccaacgatca aggcgagtta catgatcccc catgttgtgc aaaaaagcgg ttagctcctt 9420
   cggtcctccg atcgttgtca gaagtaagtt ggccgcagtg ttatcactca tggttatggc 9480
   agcactgcat aattctctta ctgtcatgcc atccgtaaga tgcttttctg tgactggtga 9540
   gtactcaacc aagtcattct gagaatagtg tatgcggcga ccgagttgct cttgcccggc 9600
   gtcaatacgg gataataccg cgccacatag cagaacttta aaagtgctca tcattggaaa 9660
   acgttcttcg gggcgaaaac tctcaaggat cttaccgctg ttgagatcca gttcgatgta 9720
   acccactcgt gcacccaact gatcttcagc atcttttact ttcaccagcg tttctgggtg 9780
   agcaaaaaca ggaaggcaaa atgccgcaaa aaagggaata agggcgacac ggaaatgttg 9840
   aatactcata ctcttccttt ttcaatatta ttgaagcatt tatcagggtt attgtctcat 9900
   gagcggatac atatttgaat gtatttagaa aaataaacaa ataggggttc cgcgcacatt 9960
   tccccgaaaa gtgccacctg acgtctaaga aaccattatt atcatgacat taacctataa 10020
   aaataggcgt atcacgaggc cctttcgtct cgcgcgtttc ggtgatgacg gtgaaaacct 10080
   ctgacacatg cagctcccgg agacggtcac agcttgtctg taagcggatg ccgggagcag 10140
   acaagcccgt cagggcgcgt cagcgggtgt tggcgggtgt cggggctggc ttaactatgc 10200
   ggcatcagag cagattgtac tgagagtgca ccatatggac atattgtcgt tagaacgcgg  10260
   ctacaattaa tacataacct tatgtatcat acacatacga tttaggtgac actatagaac  10320
   tcgagcagct g                                                       10331